# EUROPEAN PATENT APPLICATION

(11) **EP 2 901 939 A1**
(43) Date of publication of application: **05.08.2015**
(21) Application number: 15153024.3
(22) Date of filing: 29.01.2015
(51) Int. Cl.: A61B 8/08, A61B 8/04, A61B 8/00

(54) **Ultrasonic measurement apparatus and ultrasonic measurement method**

(30) Priority: 31.01.2014 JP 2014016641; 28.05.2014 JP 2014109853; 28.05.2014 JP 2014109854; 20.06.2014 JP 2014127006
(71) Applicant: Seiko Epson Corporation, Tokyo 163 (JP)
(72) Inventor: Mano, Tomonori, Nagano 392-8502 (JP); Tsukiashi, Norio, Nagano 392-8502 (JP)
(74) Representative: Hoffmann Eitle

(57) **Abstract**

A transmission and reception control unit controls transmission of an ultrasonic wave toward a blood vessel and reception of a reflected wave. A setting unit sets a signal range including a signal part for tracking movement of blood vessel walls of the blood vessel due to pulsation, and a signal waveform template in the signal range, among received signals of the reflected waves obtained at a first timing. Then, a detection unit detects a second timing when a signal waveform of the signal part corresponding to the signal range among the received signals and the signal waveform template satisfy predetermined equivalent conditions. A tracking unit starts tracking at the second timing.

## Description

### BACKGROUND

As an example of measuring biological information with an ultrasonic measurement apparatus, the evaluation of a vascular function or the determination of a vascular disease is performed. When a blood vessel is to be measured in both cases, a change in a blood vessel diameter due to pulsation is measured, that is, temporal displacement of a blood vessel wall is measured by tracking the position of the blood vessel wall, in many cases. A tracking technology for the displacement part is generally called "tracking".

As a method of tracking, an echo tracking method based on an image acquired by ultrasonic wave transmission and reception (for example, see JP-A-2007-68731) or a phase difference tracking method based on phase information of a received or transmitted ultrasonic wave signal (for example, see JP-A-10-5226) has been known.

It is necessary to designate a part to be a tracking target before starting the tracking, in both cases using any tracking method. However, according to the methods of the related art, a desired part to be tracked and a part to actually be tracked may be different.

This will be specifically described. For example, it is assumed that an operator designates an image part of a blood vessel wall as a tracking target while watching a cross-sectional image of a biological body including a blood vessel acquired by ultrasonic wave transmission and reception, and performs an operation input of a tracking starting instruction. In this case, first, the blood vessel wall is displaced due to pulsation, and accordingly, the designation of the tracking target may be difficult. Next, even when the image part of the blood vessel wall is designated, a difference in time occurs until the tracking is actually started, and therefore, the tracking may be performed for an image part different from the designated image part, as a target. These problems are particularly generated in a case of designating a fine local part in the blood vessel wall and measuring displacement of the local part with high accuracy.

A method of storing the image obtained by the ultrasonic wave transmission and reception as a moving image and tracking the part afterward by playing back the moving image using pause, has been considered, however, it is difficult to use this method when tracking in real time.

Regarding a reference beam and a related beam among a plurality of ultrasonic wave beams, JP-A-2007-68731 discloses a technology of diverting and setting a position on the related beam corresponding to a tracking point set on the reference beam, as a tracking point, based on a correlation between echo signals. However, it is clear that the above-mentioned problems are generated in the setting of the tracking point on the reference beam.

The blood vessel diameter increases and decreases due to the pulsation. Accordingly, when the designation timing of the tracking target is in a period of the large displacement of the blood vessel wall, the above-mentioned problems are more significantly generated.

### SUMMARY

A first aspect of the invention relates to an ultrasonic measurement apparatus including: a transmission and reception control unit which controls transmission of an ultrasonic wave toward a blood vessel and reception of a reflected wave; a setting unit which sets a signal range including a signal part for tracking movement of blood vessel walls of the blood vessel due to pulsation, and a signal waveform template in the signal range, among received signals of the reflected waves obtained at a first timing; a detection unit which detects a second timing when a signal waveform of the signal part corresponding to the signal range among the received signals and the signal waveform template satisfy predetermined equivalent conditions; and a tracking unit which starts the tracking at the second timing.

A second aspect of the invention relates to an ultrasonic measurement apparatus including: a transmission and reception unit which transmits an ultrasonic wave toward a blood vessel and receives the reflected ultrasonic wave as a reflected wave; a setting unit which sets a signal range including a signal relating to movement of blood vessel walls of the blood vessel, among received signals of the reflected waves obtained at a first timing; a detection unit which detects a timing when the received signals of the reflected waves satisfying predetermined conditions with the signals included in the signal range at the first timing are obtained, as a second timing; and a tracking unit which starts tracking of blood vessel walls of the blood vessel at the second timing.

A third aspect of the invention relates to an ultrasonic measurement apparatus including: a transmission and reception unit which transmits an ultrasonic wave toward a blood vessel and receives the reflected ultrasonic wave as a reflected wave; a timing detection unit which detects a first timing corresponding to diastole of the blood vessel; a setting unit which sets a signal waveform template of a signal relating to movement of blood vessel walls of the blood vessel, among received signals of the reflected waves obtained at a first timing; a detection unit which detects a timing when the signal waveform and the signal waveform template of the signal included in the received signals of the reflected waves satisfy predetermined conditions and the received signals of the reflected waves are obtained, as a second timing; and a tracking unit which starts tracking of blood vessel walls of the blood vessel at the second timing.

A fourth aspect of the invention relates to an ultrasonic measurement apparatus including: a transmission and reception control unit which controls transmission of an ultrasonic wave toward a blood vessel and reception of a reflected wave; a setting unit which sets a signal waveform template showing a part of a blood vessel wall of the blood vessel based on received signals of the reflected waves; and a tracking starting control unit which sets a tracking point based on a signal part conformable to the signal waveform template among the received signals and starts tracking.

### BRIEF DESCRIPTION OF THE DRAWINGS

The invention will be described with reference to the accompanying drawings, wherein like numbers reference like elements.
Fig. 1 is a diagram showing an example of the system configuration of an ultrasonic measurement apparatus of a first embodiment.
Figs. 2A to 2F are diagrams for illustrating a principle of a first embodiment.
Figs. 3A to 3F are diagrams for illustrating a principle of a first embodiment.
Fig. 4 is a diagram showing a functional configuration example of an ultrasonic measurement apparatus of a first embodiment.
Fig. 5 is a diagram showing a specific example of setting of a signal range and a signal waveform template.
Fig. 6 is a diagram showing a configuration example of a storage unit of a first embodiment.
Fig. 7 is a flowchart showing a flow of a measurement process of a first embodiment.
Fig. 8 is a diagram showing an example of the system configuration of an ultrasonic measurement apparatus of a second embodiment.
Figs. 9A to 9F are diagrams for illustrating a principle of a second embodiment.
Figs. 10A to 10F are diagrams for illustrating a principle of a second embodiment.
Fig. 11 is a diagram showing a functional configuration example of an ultrasonic measurement apparatus of a second embodiment.
Fig. 12 is a diagram showing a specific example of setting of a signal range and an average amplitude reference value.
Fig. 13 is a diagram showing a configuration example of a storage unit of a second embodiment.
Fig. 14 is a flowchart showing a flow of a measurement process of a second embodiment.
Fig. 15 is a diagram showing an example of the system configuration of an ultrasonic measurement apparatus of a third embodiment.
Figs. 16A to 16F are diagrams for illustrating a principle of a third embodiment.
Figs. 17A to 17F are diagrams for illustrating a principle of a third embodiment.
Fig. 18 is a flowchart showing an example of a flow of a process of detecting a first timing.
Fig. 19 is a flowchart showing an example of a flow of a process of detecting a first timing.
Fig. 20 is a flowchart showing an example of a flow of a process of detecting a first timing.
Fig. 21 is a flowchart showing an example of a flow of a process of detecting a first timing.
Fig. 22 is a diagram showing a functional configuration example of an ultrasonic measurement apparatus of a third embodiment.
Fig. 23 is a diagram showing a specific example of setting of a signal range and a signal waveform template.
Fig. 24 is a diagram showing a configuration example of a storage unit of a third embodiment.
Fig. 25 is a flowchart showing a flow of a measurement process of a third embodiment.
Fig. 26 is a diagram showing an example of the system configuration of an ultrasonic measurement apparatus of a fourth embodiment.
Figs. 27A to 27C are diagrams for schematically illustrating reflected wave data.
Fig. 28 is a diagram for illustrating initial setting of a tracking point.
Fig. 29 is a diagram showing a functional configuration example of an ultrasonic measurement apparatus of a fourth embodiment.
Fig. 30 is a diagram showing a specific example of setting of a signal waveform template.
Fig. 31 is a diagram showing a configuration example of a storage unit of a fourth embodiment.
Fig. 32 is a flowchart showing a flow of a measurement process of a fourth embodiment.

### DESCRIPTION OF EXEMPLARY EMBODIMENTS

A first feature of the invention is directed to an ultrasonic measurement apparatus including: a transmission and reception control unit which controls transmission of an ultrasonic wave toward a blood vessel and reception of a reflected wave; a setting unit which sets a signal range including a signal part for tracking movement of blood vessel walls of the blood vessel due to pulsation, and a signal waveform template in the signal range, among received signals of the reflected waves obtained at a first timing; a detection unit which detects a second timing when a signal waveform of the signal part corresponding to the signal range among the received signals and the signal waveform template satisfy predetermined equivalent conditions; and a tracking unit which starts the tracking at the second timing.

As another feature of the invention, the first feature of the invention may be configured as an ultrasonic measurement method including: controlling transmission of an ultrasonic wave toward a blood vessel and reception of a reflected wave; setting a signal range including a signal part for tracking movement of blood vessel walls of the blood vessel due to pulsation, and a signal waveform template in the signal range, among received signals of the reflected waves obtained at a first timing; detecting a second timing when a signal waveform of the signal part corresponding to the signal range among the received signals and the signal waveform template satisfy predetermined equivalent conditions; and starting the tracking at the second timing.

According to the first feature of the invention and the like, the signal range including the signal part for tracking the movement of the blood vessel walls of the blood vessel due to the pulsation, and the signal waveform template in the signal range are set among the received signals of the reflected waves of the ultrasonic waves transmitted toward the blood vessel. This setting is performed based on the received signals obtained at the first timing. Thus, the setting can be performed based on the received signal at a certain time, as a still image, and therefore it is possible to accurately set a desired tracking target.

After the setting of the tracking target, the second timing when the signal waveform of the signal part corresponding to the set signal range and the set signal waveform template among the received signals satisfy the predetermined equivalent conditions, is detected. The tracking is started at the second timing. Accordingly, although there is a difference in time between the first timing and the second timing, the part desired to be tracked and the part to actually be tracked are not different from each other.

A second feature of the invention is directed to the ultrasonic measurement apparatus according to the first feature of the invention, wherein the setting unit sets a front wall signal range and a front wall signal waveform template relating to a signal part of a front wall of the blood vessel, and a rear wall signal range and a rear wall signal waveform template relating to a signal part of a rear wall, and the detection unit detects a timing when a signal waveform of the signal part corresponding to the front wall signal range and the front wall signal waveform template satisfy the equivalent conditions and a signal waveform of the signal part corresponding to the rear wall signal range and the rear wall signal waveform template satisfy the equivalent conditions, as the second timing.

According to the second feature of the invention, the technology of the first feature of the invention is applied to the front wall and the rear wall of the blood vessel, and the timing when the front wall satisfies the equivalent conditions and the rear wall also satisfies the equivalent conditions is set as the second timing. A displacement amount of the front wall and a displacement amount of the rear wall accompanied with the pulsation may be different from each other depending on a blood vessel to be subjected to ultrasonic measurement, a position to be measured, or a subject. However, even when the displacement amount of one of the front wall and the rear wall is small, the displacement amount of the other one is great. Therefore, according to the second feature of the invention, by applying the technology of the first feature of the invention to the front wall and the rear wall, it is possible to improve accuracy of the coincidence of the desired part to be tracked and the part to actually be tracked.

A fourth feature of the invention is directed to an ultrasonic measurement apparatus including: a transmission and reception unit which transmits an ultrasonic wave toward a blood vessel and receives the reflected ultrasonic wave as a reflected wave; a setting unit which sets a signal range including a signal relating to movement of blood vessel walls of the blood vessel, among received signals of the reflected waves obtained at a first timing; a detection unit which detects a timing when the received signals of the reflected waves satisfying predetermined conditions with the signals included in the signal range at the first timing are obtained, as a second timing; and a tracking unit which starts tracking of blood vessel walls of the blood vessel at the second timing.

As another feature of the invention, the fourth feature of the invention may be configured as an ultrasonic measurement method including: transmitting an ultrasonic wave toward a blood vessel and receiving the reflected ultrasonic wave; setting a signal range including a signal relating to movement of blood vessel walls of the blood vessel, among received signals of the reflected waves obtained at a first timing; detecting a timing when the received signals of the reflected waves satisfying predetermined conditions with the signals included in the signal range at the first timing are obtained, as a second timing; and starting tracking of blood vessel walls of the blood vessel at the second timing.

According to the fourth feature of the invention and the like, the signal range including the signal for tracking the movement of the blood vessel walls of the blood vessel due to the pulsation is set among the received signals of the reflected waves of the ultrasonic waves transmitted toward the blood vessel. This setting is performed based on the received signals obtained at the first timing. Thus, the setting can be performed based on the received signal at a certain time, as a still image, and therefore it is possible to accurately set a desired tracking target.

After the setting of the tracking target, the second timing when the signal corresponding to the set signal range satisfies the predetermined conditions with the signal in the signal range at the first timing, is detected. The tracking is started at the second timing. Accordingly, although there is a difference in time between the first timing and the second timing, the part desired to be tracked and the part to actually be tracked are not different from each other.

A fifth feature of the invention is directed to the ultrasonic measurement apparatus according to the fourth of the invention, wherein the setting unit sets a front wall signal range relating to a signal of a front wall of the blood vessel and a rear wall signal range relating to a signal of a rear wall, and the detection unit detects a timing when the signal corresponding to the front wall signal range satisfies the conditions and the signal corresponding to the rear wall signal range satisfies the conditions, as a second timing.

According to the fifth feature of the invention, the technology of the fourth feature of the invention is applied to the front wall and the rear wall of the blood vessel, and the timing when the front wall satisfies the conditions and the rear wall also satisfies the conditions is set as the second timing. A displacement amount of the front wall and a displacement amount of the rear wall accompanied with the pulsation may be different from each other depending on a blood vessel to be subjected to ultrasonic measurement, a position to be measured, or a subject. However, even when the displacement amount of one of the front wall and the rear wall is small, the displacement amount of the other one is great. Therefore, according to the second feature of the invention, by applying the technology of the fourth feature of the invention to the front wall and the rear wall, it is possible to improve accuracy of the coincidence of the desired part to be tracked and the part to actually be tracked.

A sixth feature of the invention is directed to the ultrasonic measurement apparatus according to the fourth feature of the invention, wherein the predetermined conditions are conditions based on any one of a total amplitude value, an average amplitude value, and a mean amplitude value of the signals corresponding to the signal ranges.

According to the sixth feature of the invention, it is possible to determine the predetermined conditions based on any one of the total amplitude value, the average amplitude value, and the mean amplitude value of the signals corresponding to the signal ranges.

An eighth feature of the invention is directed to an ultrasonic measurement apparatus including: a transmission and reception unit which transmits an ultrasonic wave toward a blood vessel and receives the reflected ultrasonic wave as a reflected wave; a timing detection unit which detects a first timing corresponding to diastole of the blood vessel; a setting unit which sets a signal waveform template of a signal relating to movement of blood vessel walls of the blood vessel, among received signals of the reflected waves obtained at a first timing; a detection unit which detects a timing when the signal waveform and the signal waveform template of the signal included in the received signals of the reflected waves satisfy predetermined conditions and the received signals of the reflected waves are obtained, as a second timing; and a tracking unit which starts tracking of blood vessel walls of the blood vessel at the second timing.

As another feature of the invention, the eighth aspect of the invention may be configured as an ultrasonic measurement method including: transmitting an ultrasonic wave toward a blood vessel and receiving the reflected ultrasonic wave; detecting a first timing corresponding to diastole of the blood vessel; setting a signal waveform template of a signal relating to movement of blood vessel walls of the blood vessel, among received signals of the reflected waves obtained at a first timing; detecting a timing when the signal waveform and the signal waveform template of the signal included in the received signals of the reflected waves satisfy predetermined conditions and the received signals of the reflected waves are obtained, as a second timing; and starting tracking of the blood vessel wall at the second timing.

According to the eighth feature of the invention and the like, the signal waveform template of the signal relating to the movement of the blood vessel walls of the blood vessel is set among the received signals of the reflected waves of the ultrasonic waves transmitted toward the blood vessel. This setting is performed based on the received signals obtained at the first timing. Thus, the setting can be performed based on the received signal at a certain time, as a still image, and therefore it is possible to accurately set a desired tracking target.

After the setting of the tracking target, the second timing when the signal waveform of the received signal and the set signal waveform template satisfy the conditions, is detected. The tracking is started at the second timing. In addition, the first timing is during diastole when a change in the position of the blood vessel wall is slight. Accordingly, the second timing is also during diastole. Accordingly, although there is a difference in time between the first timing and the second timing, a possibility of the part desired to be tracked and the part to actually be tracked being different from each other is extremely low.

A ninth feature of the invention is directed to the ultrasonic measurement apparatus according to the eighth feature of the invention, wherein the timing detection unit detects the first timing by tracking the received signals of the reflected waves in advance.

According to the ninth feature of the invention, it is possible to detect the first timing by tracking the received signals of the reflected wave of the ultrasonic wave transmitted toward the blood vessel, in advance.

A tenth feature of the invention is directed to the ultrasonic measurement apparatus according to the ninth feature of the invention, wherein the timing detection unit detects a timing when displacement of peak waveform parts included in the received signals satisfies predetermined conditions, as the first timing.

According to the tenth feature of the invention, for example, it is possible to detect the timing when displacement of the peak waveform parts included in the received signals is small, as the first timing.

An eleventh feature of the invention is directed to the ultrasonic measurement apparatus according to the tenth feature of the invention, wherein the timing detection unit detects the first timing based on the displacement of the peak waveform parts shown in a position of an external film part of the blood vessel.

According to the eleventh feature of the invention, it is possible to detect the first timing based on the displacement of the peak waveform parts shown in the position of the external film part of the blood vessel.

A twelfth feature of the invention is directed to the ultrasonic measurement apparatus according to the ninth feature of the invention, wherein the timing detection unit detects the first timing by measuring a change in a blood vessel diameter of the blood vessel by the tracking in advance.

According to the twelfth feature of the invention, it is possible to detect the first timing based on the change in the blood vessel diameter.

A thirteenth feature of the invention is directed to the ultrasonic measurement apparatus according to the eighth feature of the invention, wherein the transmission and reception unit performs the reception at a predetermined frame rate, and the timing detection unit detects a timing when correlation values obtained by a correlation operation of the received signals in preceding and succeeding frames satisfy predetermined conditions, as the first timing.

According to the thirteenth feature of the invention, it is possible to detect the first timing based on the correlation value of the received signal received in preceding and succeeding frames in time series.

A fourteenth feature of the invention is directed to the ultrasonic measurement apparatus according to the eighth feature of the invention, wherein the timing detection unit detects a timing of a telediastolic period from the diastole, as the first timing.

According to the fourteenth feature of the invention, it is possible to detect the timing of the telediastolic period as the first timing.

A fifteenth feature of the invention is directed to the ultrasonic measurement apparatus according to the eighth feature of the invention, wherein the setting unit sets a front wall signal range and a front wall signal waveform template relating to a signal part of a front wall of the blood vessel, and a rear wall signal range and a rear wall signal waveform template relating to a signal part of a rear wall, and the detection unit detects a timing when a signal waveform of the signal part corresponding to the front wall signal range and the front wall signal waveform template satisfy the conditions and a signal waveform of the signal part corresponding to the rear wall signal range and the rear wall signal waveform template satisfy the conditions, as the second timing.

According to the fifteenth feature of the invention, the technology of the eighth feature of the invention is applied to the front wall and the rear wall of the blood vessel, and the timing when the front wall satisfies the conditions and the rear wall also satisfies the conditions is set as the second timing. A displacement amount of the front wall and a displacement amount of the rear wall accompanied with the pulsation may be different from each other depending on a blood vessel to be subjected to ultrasonic measurement, a position to be measured, or a subject. However, even when the displacement amount of one of the front wall and the rear wall is small, the displacement amount of the other one is great. Therefore, according to the fifteenth feature of the invention, by applying the technology of the feature of the invention to the front wall and the rear wall, it is possible to improve accuracy of the coincidence of the desired part to be tracked and the part to actually be tracked.

A seventeenth feature of the invention is directed to an ultrasonic measurement apparatus including: a transmission and reception control unit which controls transmission of an ultrasonic wave toward a blood vessel and reception of a reflected wave; a setting unit which sets a signal waveform template showing a part of a blood vessel wall of the blood vessel based on received signals of the reflected waves; and a tracking starting control unit which sets a tracking point based on a signal part conformable to the signal waveform template among the received signals and starts tracking.

As another feature of the invention, the seventeenth feature of the invention may be configured as an ultrasonic measurement method including: controlling transmission of an ultrasonic wave toward a blood vessel and reception of a reflected wave; setting a signal waveform template showing a part of a blood vessel wall of the blood vessel based on received signals of the reflected waves; and setting a tracking point based on a signal part conformable to the signal waveform template among the received signals and starting tracking.

According to the seventeenth feature of the invention and the like, the signal waveform template showing the part of the blood vessel wall of the blood vessel is set among the received signals of the reflected waves of the ultrasonic waves transmitted toward the blood vessel. This setting can be performed based on the received signal obtained at an arbitrary timing. Thus, the setting can be performed based on the received signal at a certain time, as a still image, and therefore it is possible to accurately set the signal waveform template relating to the desired tracking target.

After the setting of the signal waveform template, the tracking point is set based on the signal part conformable to the set signal waveform template among the received signals, and the tracking is started. Accordingly, although there is a difference in time from the timing when the signal waveform template is set to the timing when the tracking is actually started, the part desired to be tracked and the part to actually be tracked are not different from each other.

An eighteenth feature of the invention is directed to the ultrasonic measurement apparatus according to the seventeenth feature of the invention, wherein the tracking starting control unit includes a searching unit which performs a predetermined correlation operation and searches a signal part where a correlation value of the signal part and the signal waveform template satisfies predetermined conformance conditions, and sets the tracking point based on the searched signal part.

According to the eighteenth feature of the invention, by performing the predetermined correlation operation, the signal part where the correlation value among the received signals satisfies the predetermined conformance conditions, as the signal part conformable to the signal waveform template, is searched. Therefore, it is possible to improve accuracy of the coincidence of the desired part to be tracked and the part to actually be tracked.

A nineteenth feature of the invention is directed to an ultrasonic measurement apparatus comprising a transmission and reception control unit which transmits an ultrasonic wave toward a blood vessel and receives a reflected wave; a setting unit which sets a signal waveform template showing a part of a blood vessel wall of the blood vessel based on received signals of the reflected waves; and a tracking starting control unit which sets a tracking point based on a signal part conformable to the signal waveform template among the received signals and starts tracking.

A twentieth feature of the invention is directed to the ultrasonic measurement apparatus according to the nineteenth feature of the invention, wherein the tracking starting control unit includes a searching unit which performs a predetermined correlation operation and searches a signal part where a correlation value of the signal part and the signal waveform template satisfies predetermined conformance conditions, and sets the tracking point based on the searched signal part.

A twenty-first feature of the invention is directed to an ultrasonic measurement method comprising controlling transmission of an ultrasonic wave toward a blood vessel and reception of a reflected wave; setting a signal waveform template showing a part of a blood vessel of the blood vessel based on received signals of the reflected waves; and setting a tracking point based on a signal part conformable to the signal waveform template among the received signals and starting tracking.

In the ultrasonic measurement apparatus according to the first, fifth, eighth, and seventeenth, nineteenth and twentieth features of the invention, the blood vessel to be measured may be an artery.

### Embodiments

Hereinafter, preferred embodiments of the invention will be described, but the applicable embodiments of the invention are not limited to the following embodiments.

### First Embodiment

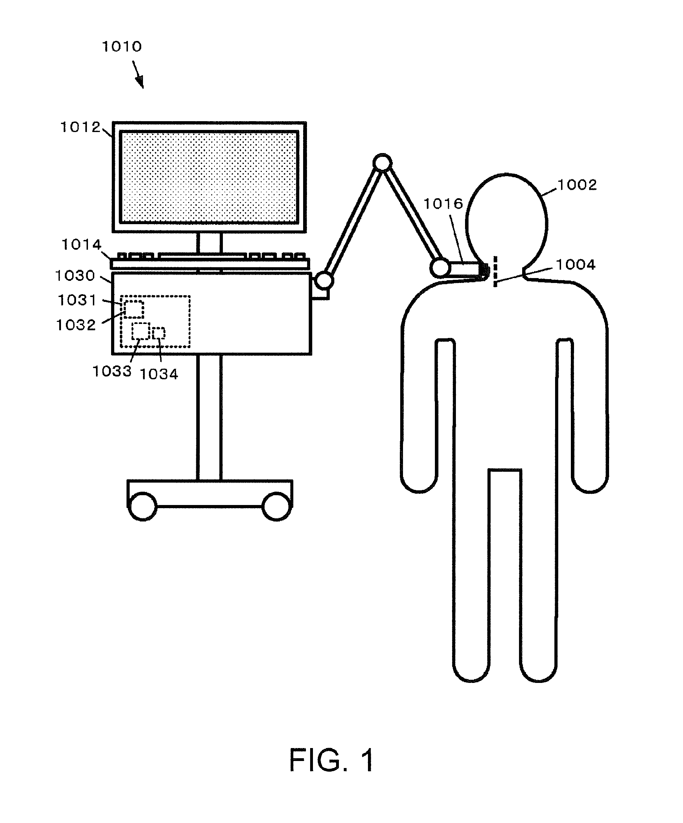

Fig. 1 is a diagram showing an example of the system configuration of an ultrasonic measurement apparatus 1010 according to a first embodiment. The ultrasonic measurement apparatus 1010 is an apparatus that measures biological information of a subject 1002 by measuring the reflected waves of ultrasonic waves. In the first embodiment, vascular function information such as blood pressure relating to a blood vessel 1004 which is an artery and an intima media thickness (IMT), is measured as a piece of biological information. In Fig. 1, a carotid artery is set as a blood vessel 1004 to be measured, but another artery such as a radial artery may be set as the blood vessel 1004 to be measured.

The ultrasonic measurement apparatus 1010 includes a touch panel 1012 serving as a unit that displays a measurement result or operation information as an image and as an operation input unit, a keyboard 1014 used for operation input, an ultrasonic probe 1016, and a processor 1030. A control board 1031 is mounted in the processor 1030, and is connected to each unit of the apparatus, such as the touch panel 1012, the keyboard 1014, and the ultrasonic probe 1016, so that signal transmission and reception therebetween are possible.

Not only various integrated circuits, such as a central processing unit (CPU) 1032 and an application specific integrated circuit (ASIC), but also a storage medium 1033, such as an IC memory or a hard disk, and a communication IC 1034 for realizing data communication with an external device are mounted on the control board 1031. The processor 1030 realizes various functions according to the first embodiment such as a control operation for starting tracking of a blood vessel wall of the blood vessel 1004 according to the ultrasonic measurement and measurement of vascular function information for the blood vessel 1004 by executing a measurement program stored in the storage medium 1033 with the CPU 1032 or the like.

Specifically, by the control of the processor 1030, the ultrasonic measurement apparatus 1010 transmits and emits an ultrasonic beam from the ultrasonic probe 1016 to the subject 1002 and receives the reflected wave. Then, by performing signal processing on a received signal of the reflected wave (hereinafter, simply referred to as a "received signal"), it is possible to generate reflected wave data, such as a temporal change or position information of a structure in the living body of the subject 1002. Images in respective modes of so-called A mode, B mode, M mode, and color Doppler are included in the reflected wave data. Measurement using an ultrasonic wave is repeatedly performed at predetermined periods. The measurement unit is referred to as a "frame".

By setting a tracking point (a region of interest) in the received signal as a reference, the ultrasonic measurement apparatus 1010 can perform so-called "tracking" that is tracking the tracking point in time series between different frames and calculating the displacement. In the first embodiment, a front wall and a rear wall of the blood vessel 1004 are set as the tracking points, and the ultrasonic measurement apparatus 1010 has characteristics relating to the setting and starting control of the tracking points.

### Description of Principle of First Embodiment

Principles of the setting of the tracking points and the starting control in the first embodiment will be described.

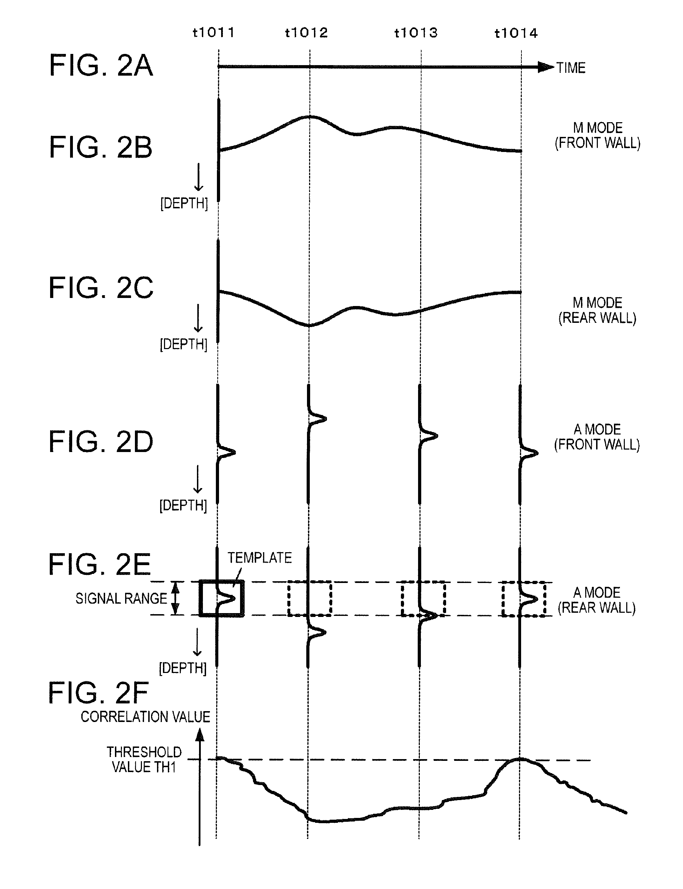

Figs. 2A to 2F are diagrams schematically showing the reflected wave data obtained by signal processing of the received data, in which Fig. 2A shows a time axis, Fig. 2B shows a schematic M mode image relating to the front wall, Fig. 2C shows a schematic M mode image relating to the rear wall, Fig. 2D shows a schematic A mode image relating to the front wall, and Fig. 2E shows a schematic A mode image relating to the rear wall. The A mode image can be referred to as the received signal as it is, and therefore, the A mode image will be described as having the same meaning as the received signal, hereinafter. In the ultrasonic measurement, the reflected wave from the front wall and the rear wall of the blood vessel 1004 is detected strongly. The signal parts of the received signal shown in Figs. 2D and 2E are obtained by cutting out a circumferential part including the signal parts from the front wall and the rear wall, and are signal parts in a constant depth range.

The front wall and the rear wall of the blood vessel 1004 are displaced back and forth when seen from the ultrasonic probe 1016 (in depth direction) due to the pulsation. Accordingly, the part of the front wall and the part of the rear wall among the reflected wave signals are displaced as shown in Figs. 2B to 2E. Time points t1011 to t1014 are for one pulse. Herein, the front wall and the rear wall are displaced, but return to the same position after the one pulse. For example, in Fig. 2E, peaks of the rear walls are shown in substantially the same depth position in a signal waveform at the time point t1011 and in a signal waveform at the time point t1014. In the first embodiment, the starting control of the tracking is performed according to the above description.

First, a target desired to be tracked is designated and set from the received signal obtained at the first timing. It is possible to set a tracking target from the received signals obtained at the first timing as a so-called still image, and accordingly, it is possible to precisely set the tracking target by magnifying the received signal, for example. Fig. 2E shows an example in which the rear wall is set as a tracking target. The time point t1011 is the first timing. Among the received signals at the time point t1011, a depth range including the signal part of the rear wall is set as a signal range (depth range) desired to be the tracking target. This signal range is a region surrounded with a thick line. A signal waveform in the signal range is set as a template waveform.

When the setting of the signal range is completed, hereinafter, the acquired received signal is continuously monitored in real time. Specifically, a correlation operation of the signal waveform in the signal range among the received signals and the template waveform is repeatedly performed, and a second timing, at which a correlation value satisfies threshold value conditions is detected. That is, the second timing at which equivalent conditions in which the signal waveform in the signal range among the received signals corresponds to the template waveform are satisfied, is continuously monitored. The tracking is started at this second timing.

Fig. 2F shows a change in the correlation value. A timing at which the correlation value is equal to or greater than a threshold value is detected as the second timing. The time point t1014 is the second timing. As shown in Fig. 2E, at the time point t1014, the rear wall is positioned in the same depth position as the time point t1011 which is the first timing. Accordingly, the part desired to be tracked and the part to actually be tracked are not different from each other. Since the tracking is started from the received signal acquired at the second timing (time point t1014), the tracking point (region of interest) is set from the received signal acquired at the second timing. Specifically, the tracking is started by using the signal part of the signal range as the tracking point (region of interest), among the received signals acquired at the second timing.

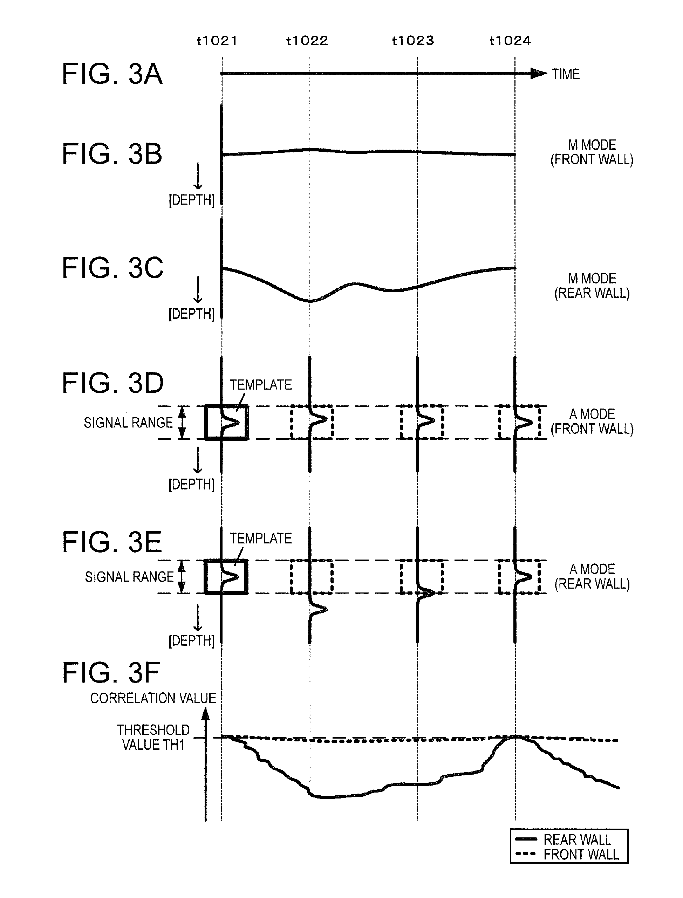

The tracking target may be only one of the front wall and the rear wall, but both walls are designated and set in the first embodiment. This is because a displacement amount of one of the front wall and the rear wall may be great, while a displacement amount of the other one is small. This phenomenon may occur due to a vascular state or vascular surrounding tissues and may occur depending on a blood vessel to be measured, a position to be measured, or a subject. Figs. 3A to 3F show an example of a case in which the displacement amount of one of the front wall and the rear wall is great and the displacement amount of the other one is small. Figs. 3A to 3F show a case in which the displacement amount of the front wall is small and the displacement amount of the rear wall is great. Figs. 3A to 3F are shown in the same manner as Figs. 2A to 2F.

In Figs. 3A to 3F, the depth positions of the front wall are substantially the same positions regardless of the pulsation. Accordingly, as shown in Fig. 3F, the correlation value relating to the front wall is a substantially constant value, and the second timing at which the correlation value is equal to or greater than a threshold value TH1 may be before a time point t1024. By designating and setting of both the front wall and the rear wall as the tracking targets, it is possible to more accurately detect the second timing.

### Description of Functional Configuration of First Embodiment

Next, the functional configuration for realizing the first embodiment will be described.

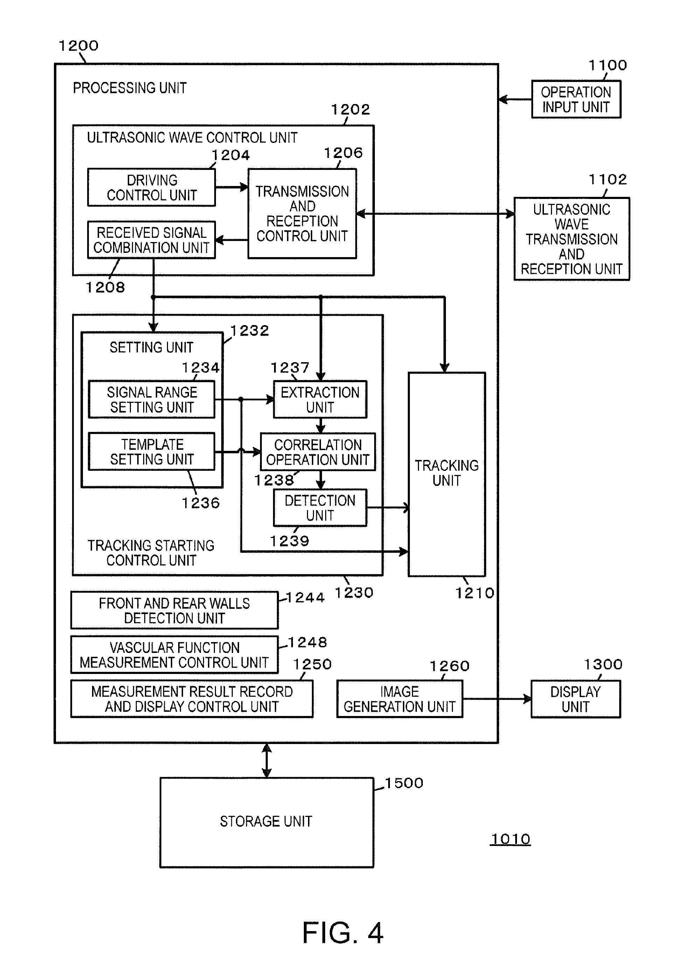

Fig. 4 is a block diagram showing an example of the functional configuration of the ultrasonic measurement apparatus 1010 in the first embodiment.

The ultrasonic measurement apparatus 1010 includes an operation input unit 1100, an ultrasonic wave transmission and reception unit 1102, a processing unit 1200, a display unit 1300, and a storage unit 1500.

The operation input unit 1100 receives various kinds of operation input by the operator, and outputs an operation input signal corresponding to the operation input to the processing unit 1200. The operation input unit 1100 can be implemented by a button switch, a lever switch, a dial switch, a track pad, a mouse, or the like. In the example shown in Fig. 1, the touch panel 1012 or the keyboard 1014 corresponds to the operation input unit.

The ultrasonic wave transmission and reception unit 1102 transmits an ultrasonic wave with a pulse voltage output from the processing unit 1200. Then, the ultrasonic wave transmission and reception unit receives a reflected wave of the transmitted ultrasonic wave, converts the reflected wave into an electrical signal, and outputs the electrical signal to the processing unit 1200. The ultrasonic probe 1016 shown in Fig. 1 corresponds to the ultrasonic wave transmission and reception unit.

The processing unit 1200 is realized by a microprocessor, such as a CPU or a graphic processor unit (GPU), or an electronic component, such as an ASIC, a field-programmable gate array (FPGA) or an integrated circuit (IC) memory, for example. In addition, the processing unit 1200 performs control of the input and output of data to each functional unit, and calculates biological information of the subject 1002 by performing various kinds of arithmetic processing based on a predetermined program or data, the operation input signal from the operation input unit 1100, the signal from the ultrasonic wave transmission and reception unit 1102, or the like. The processor 1030 and the control board 1031 shown in Fig. 1 correspond to the processing unit 1200.

The processing unit 1200 includes an ultrasonic wave control unit 1202, a tracking unit 1210, a tracking starting control unit 1230, a front and rear walls detection unit 1244, a vascular function measurement control unit 1248, a measurement result record and display control unit 1250, and an image generation unit 1260.

The ultrasonic wave control unit 1202 controls the transmission of an ultrasonic wave toward the blood vessel and the reception of a reflected wave. For example, the ultrasonic wave control unit includes a driving control unit 1204, a transmission and reception control unit 1206, and a received signal combination unit 1208, and performs overall control of ultrasonic measurement. The ultrasonic wave control unit 1202 can be realized by the well-known technologies.

The driving control unit 1204 controls the transmission timing of ultrasonic pulses from the ultrasonic probe 1016, and outputs a transmission control signal to the transmission and reception control unit 1206.

The transmission and reception control unit 1206 generates a pulse voltage according to the transmission control signal from the driving control unit 1204, and outputs the pulse voltage to the ultrasonic wave transmission and reception unit 1102. In this case, it is possible to adjust the output timing of the pulse voltage to each ultrasonic transducer by performing transmission delay processing. In addition, it is possible to perform the amplification or filtering of the signal output from the ultrasonic wave transmission and reception unit 1102 and to output the result to the received signal combination unit 1208.

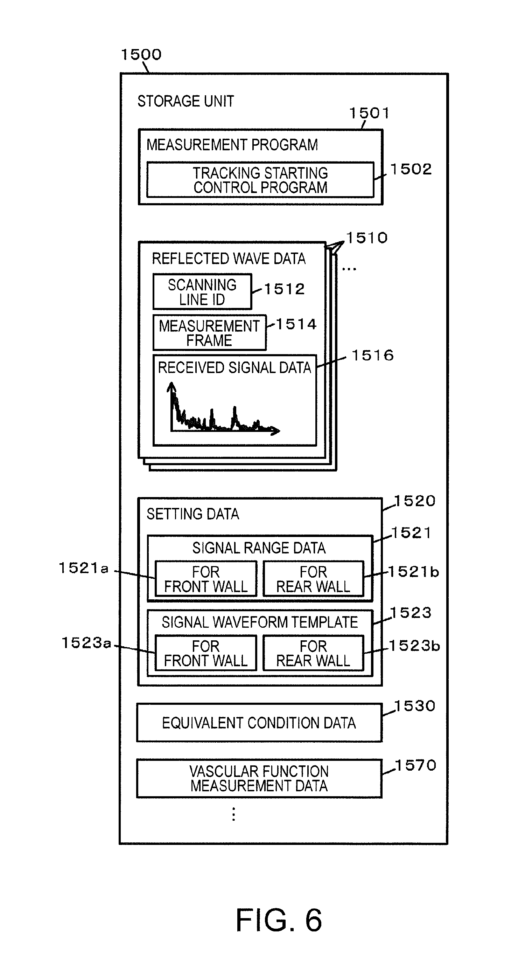

The received signal combination unit 1208 performs processing related to the so-called focus of a received signal by performing delay processing as necessary, thereby generating reflected wave data 1510 including received signal data 1516 (see Fig. 6). The reflected wave data 1510 is used in the tracking starting control unit 1230 and the tracking unit 1210.

The tracking starting control unit 1230 is a functional unit that performs starting control of the tracking described above, and includes a setting unit 1232, an extraction unit 1237, a correlation operation unit 1238, and a detection unit 1239. In the first embodiment, the tracking starting control unit 1230 is realized as the software by reading out and executing a tracking starting control program 1502 (see Fig. 6) by the processing unit 1200, but the tracking starting control unit may be realized as the hardware by the electronic circuit such as an FPGA. The extraction unit 1237, the correlation operation unit 1238, and the detection unit 1239 may be configured with the electronic circuit such as an FPGA.

The setting unit 1232 is a functional unit that sets the tracking target, and includes a signal range setting unit 1234 that sets a position range (signal range) in a depth direction in the received signal according to the operation input of the operation input unit 1100, and a template setting unit 1236 that sets a signal waveform in the signal range in the received signal as a signal waveform template. The signal range setting unit 1234 respectively sets signal ranges for the front wall and the rear wall and stores front wall signal range data 1521a and rear wall signal range data 1521b (collectively referred to as "signal range data 1521") in the storage unit 1500 (see Fig. 6). In the same manner as described above, the template setting unit 1236 also respectively sets signal waveform templates for the front wall and the rear wall and stores a front wall signal waveform template 1523a and a rear wall signal waveform template 1523b (collectively referred to as a "signal waveform template 1523") in the storage unit 1500.

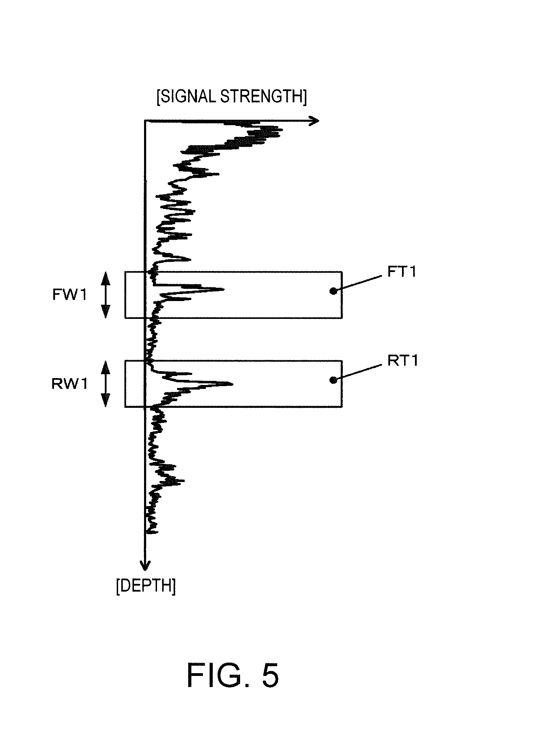

The setting of the signal range and the signal waveform template will be described in detail with reference to Fig. 5. Fig. 5 shows an example of a reflected wave signal obtained at a certain timing (first timing). The setting unit 1232, for example, displays the received signal on the display unit 1300 and sets the front wall signal range FW1 (1521a) and the rear wall signal range RW1 (1521b) according to the setting instruction operation from the operation input unit 1100. The signal ranges (front wall signal range FW1 and rear wall signal range RW1) are data for determining a range of the depth position. When setting the signal ranges, it is possible to accurately set the ranges by magnifying and displaying the received signal.

When the setting of the signal range is completed, the setting unit 1232 cuts out a signal part in the front wall signal range FW1 and sets the signal part as the front wall signal waveform template FT1 (1523a), and cuts out a signal part in the rear wall signal range RW1 and sets the signal part as the rear wall signal waveform template RT1 (1523b).

The description will be performed by returning to Fig. 4. The extraction unit 1237, the correlation operation unit 1238, and the detection unit 1239 function to detect the second timing.

The extraction unit 1237 extracts the signal part in the front wall signal range FW1 and the signal part in the rear wall signal range RW1 among the received signals output from the ultrasonic wave control unit 1202 on each occasion, and outputs the signal parts to the correlation operation unit 1238.

For the front wall and the rear wall, the correlation operation unit 1238 performs the correlation operation of the signal waveform templates and the signal parts extracted by the extraction unit 1237, calculates a correlation value, and outputs the correlation value to the detection unit 1239. As the correlation operation, normalized cross-correlation or zero-mean normalized cross-correlation can be used, for example.

The detection unit 1239 detects whether or not the correlation value satisfies the equivalent conditions, that is, whether or not the correlation value is equal to or greater than the threshold value, thereby detecting the second timing. When the second timing is detected, the detection unit outputs a tracking starting instruction signal to the tracking unit 1210.

The tracking unit 1210 starts the tracking according to the tracking starting instruction signal from the detection unit 1239. The tracking points (regions of interest) are set in the signal parts in the signal ranges among the received signals at the time when the tracking starting instruction signal is input. Accordingly, the parts desired to be tracked are accurately set as the tracking points. For the tracking hereinafter, the well-known technologies can be used. For example, the functions of the "echo tracking" or the "phase difference tracking" are realized.

The front and rear walls detection unit 1244 detects vascular feature points from the received signal, as a previous step of the setting by the setting unit 1232. Specifically, two peaks which have signal strengths equal to or greater than a predetermined signal strength and in which the signal strength therebetween is equal to or smaller than a predetermined low strength indicating blood, are detected as the front wall and the rear wall. The detecting method of the front wall and the rear wall is not limited to this method, and the other methods may be used.

The vascular function measurement control unit 1248 determines the displacement of the blood vessel diameter based on the positions of the front wall and the rear wall of the blood vessel 1004 which are continuously measured by the tracking unit 1210, and performs the control relevant to predetermined vascular function measurement. For example, the operation control of executing processing of estimating a blood pressure from the blood vessel diameter using a stiffness parameter and measuring the blood pressure is performed, for example.

The measurement result record and display control unit 1250 performs control for storing the measurement result of the vascular function in the storage unit 1500 and displaying the measurement result on the display unit 1300.

The image generation unit 1260 generates an image for displaying a measurement result or various operation screens required for ultrasonic measurement or biological information measurement, and outputs the image to the display unit 1300.

The display unit 1300 displays image data input from the image generation unit 1260. The touch panel 1012 shown in Fig. 1 corresponds to the display unit.

The storage unit 1500 is realized by a storage medium, such as an IC memory, a hard disk, or an optical disc, and stores various programs or various kinds of data, such as data in the operation process of the processing unit 1200. In Fig. 1, the storage medium 1033 mounted in the control board 1031 of the processor 1030 corresponds to the storage unit. In addition, the connection between the processing unit 1200 and the storage unit 1500 is not limited to a connection using an internal bus circuit in the apparatus, and may be realized by using a communication line, such as a local area network (LAN) or the Internet. In this case, the storage unit 1500 may be realized by using a separate external storage device from the ultrasonic measurement apparatus 1010.

In addition, as shown in Fig. 6, the storage unit 1500 stores a measurement program 1501, reflected wave data 1510, setting data 1520, equivalent condition data 1530, and vascular function measurement data 1570. Needless to say, frame identification information, various flags, counter values for time checking, and the like other than those described above can also be appropriately stored.

The processing unit 1200 realizes the functions of the ultrasonic wave control unit 1202, the tracking starting control unit 1230, the front and rear walls detection unit 1244, the vascular function measurement control unit 1248, the measurement result record and display control unit 1250, and the image generation unit 1260, by reading out and executing the measurement program 1501. The tracking starting control program 1502 for realizing the function of the tracking starting control unit 1230 is included in the measurement program 1501 as a subroutine program.

In addition, when realizing these functional units with hardware, such as an electronic circuit, a part of the program for realizing the function can be omitted.

The reflected wave data 1510 is reflected wave data obtained by ultrasonic measurement, and is generated for each frame by the ultrasonic wave control unit 1202. A piece of reflected wave data 1510, for example, includes a scanning line ID 1512, a measurement frame 1514, and received signal data (depth-signal strength data) 1516. Needless to say, data other than those described above can also be appropriately stored.

The setting data 1520 stores the signal range data 1521 which is data of the signal ranges set by the setting unit 1232, and the signal waveform template 1523, based on the received signal at the first timing.

The equivalent condition data 1530 is data for determining the threshold conditions of the correlation value for the detection unit 1239 to detect the second timing, and the threshold value TH1 of Figs. 2A to 3F are determined, for example.

The vascular function measurement data 1570 stores the data in which the positions of the front wall and the rear wall of the blood vessel 1004 tracked by the tracking unit 1210 correspond to the time information at which the corresponding received signal is obtained, the data of the blood vessel diameter calculated from the positions of the front wall and the rear wall, or the data of the blood pressure calculated based on the blood vessel diameter.

### Description of Flow of Process of First Embodiment

Next, the operation of the measurement process of the ultrasonic measurement apparatus 1010 will be described.

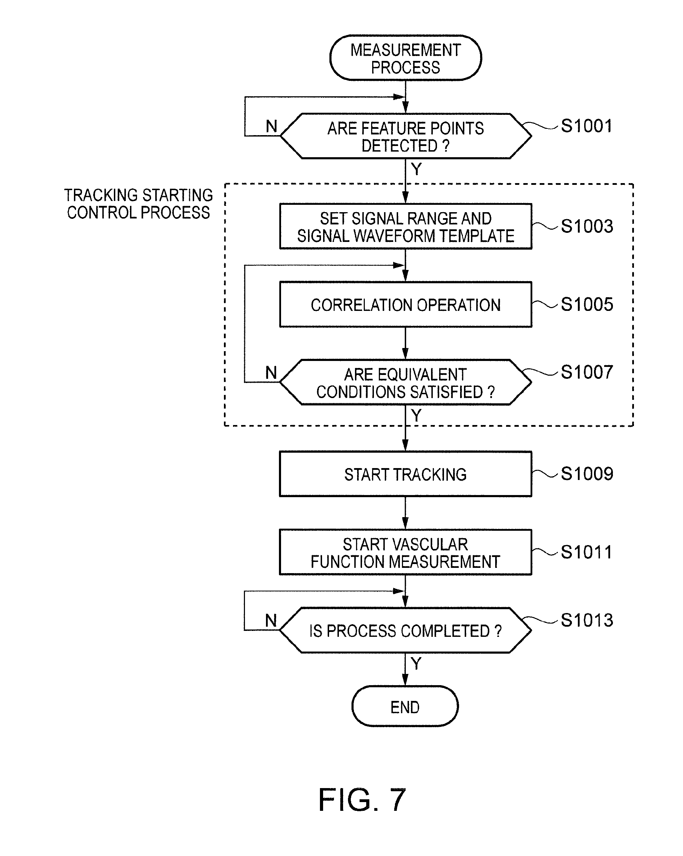

Fig. 7 is a flowchart for illustrating a flow of a measurement process of the ultrasonic measurement apparatus 1010 in the first embodiment.

In this process, the processing unit 1200 starts a process of transmitting an ultrasonic beam for each ultrasonic transducer (scanning line) of the ultrasonic wave transmission and reception unit 1102, receiving the reflected wave, and storing the reflected wave data 1510 in the storage unit 1500 on each occasion.

First, the front and rear walls detection unit 1244 determines whether or not the feature points are included in the received signal, based on the received signal data 1516 obtained by the transmission and reception of the ultrasonic waves on each occasion, and therefore stands by until the feature points are detected (Step S1001: No). In the first embodiment, the feature points are a peak indicating the front wall and the rear wall of the blood vessel 1004. When the feature points are detected (Step S1001: YES), the tracking starting control process is performed by the tracking starting control unit 1230 (Steps S1003 to S1007).

That is, the setting unit 1232 sets the signal range and the signal waveform template using the received signal obtained at the first timing (Step S1003). Then, the extraction unit 1237 extracts the signal part in the signal range among the subsequent received signals on each occasion, and the correlation operation unit 1238 determines whether or not the signal part corresponds to the signal waveform template using the equivalent condition data 1530. Specifically, the correlation operation unit 1238 performs the correlation operation of the signal part and the signal waveform template (Step S1005) and determines whether or not the correlation value satisfies the equivalent conditions (threshold value conditions), that is, the second timing (Step S1007). The processes in Step S1005 to s1007 are repeated and the second timing is monitored, until the equivalent conditions are satisfied (Step S1007: NO).

When the equivalent conditions are satisfied (Step S1007: YES), the detection unit 1239 outputs the tracking starting instruction signal to the tracking unit 1210 when the second timing is detected, and the tracking unit 1210 starts the tracking (Step S1009). The vascular function measurement control unit 1248 starts the measurement of the vascular function (Step S1011).

After that, when the completion operation of the measurement process is performed (Step S1013: YES), the measurement process ends.

Hereinabove, according to the first embodiment, the signal range including the signal part for tracking the movement of the blood vessel walls of the blood vessel due to the pulsation, and the signal waveform template in the signal range, among the received signals obtained by receiving the reflected wave of the ultrasonic wave transmitted to the blood vessel 1004, are set. This setting is performed based on the received signals obtained at the first timing. Thus, the setting can be performed based on the received signal at a certain time, as a still image, and therefore it is possible to accurately set the desired tracking target.

After the setting of the tracking target, the second timing when the signal part corresponding to the set signal range and the set signal waveform template among the received signals satisfy the predetermined equivalent conditions, is detected. The tracking is started at the second timing. Accordingly, although there is a difference in time between the first timing and the second timing, the part desired to be tracked and the part to actually be tracked are not different from each other, regardless of the problems regarding the difference in time.

In the first embodiment, it is possible to appropriately add, omit, and change the constituent elements. For example, both of the front wall and the rear wall of the blood vessel are the tracking targets in the above description, but only one thereof may be the tracking target.

In addition, the correlation operation is performed in the above description, but instead of the similarity such as the correlation value, dissimilarity such as sum of squared difference (SSD) or sum of absolute difference (SAD) may be used. However, when using SSD or SAD, as the value decreases the similarity increases, and therefore the equivalent conditions (threshold value condition) are set to have the value equal to or smaller than the threshold value.

### Second Embodiment

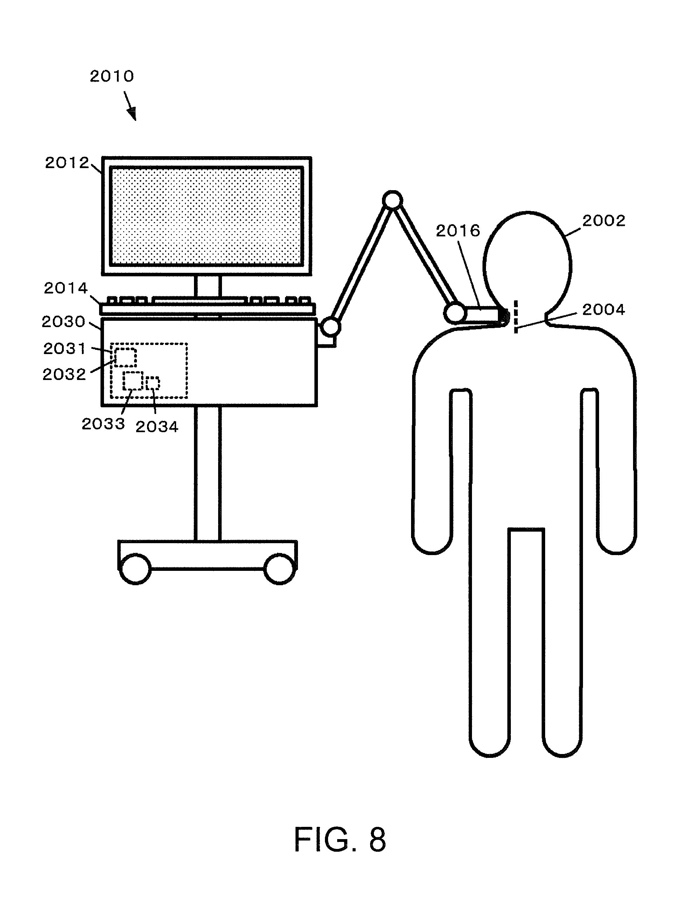

Fig. 8 is a diagram showing an example of the system configuration of an ultrasonic measurement apparatus 2010 according to a second embodiment. The ultrasonic measurement apparatus 2010 is an apparatus that measures biological information of a subject 2002 by measuring the reflected waves of ultrasonic waves. In the second embodiment, vascular function information such as blood pressure relating to a blood vessel 2004 which is an artery and an intima media thickness (IMT), is measured as a piece of biological information. In Fig. 8, a carotid artery is set as a blood vessel 2004 to be measured, but the other artery such as a radial artery may be set as the blood vessel 2004 to be measured.

The ultrasonic measurement apparatus 2010 includes a touch panel 2012 serving as a unit that displays a measurement result or operation information as an image and as an operation input unit, a keyboard 2014 used for operation input, an ultrasonic probe 2016, and a processor 2030. A control board 2031 is mounted in the processor 2030, and is connected to each unit of the apparatus, such as the touch panel 2012, the keyboard 2014, and the ultrasonic probe 2016, so that signal transmission and reception therebetween are possible.

Not only various integrated circuits, such as a central processing unit (CPU) 2032 and an application specific integrated circuit (ASIC), but also a storage medium 2033, such as an IC memory or a hard disk, and a communication IC 2034 for realizing data communication with an external device are mounted on the control board 2031. The processor 2030 realizes various functions according to the second embodiment such as a control operation for starting tracking of a vessel wall of the blood vessel 2004 according to the ultrasonic measurement and measurement of vascular function information for the blood vessel 2004 by executing a measurement program stored in the storage medium 2033 with the CPU 2032 or the like.

Specifically, by the control of the processor 2030, the ultrasonic measurement apparatus 2010 transmits and emits an ultrasonic beam from the ultrasonic probe 2016 to the subject 2002 and receives the reflected wave. Then, by performing signal processing on a received signal of the reflected wave (hereinafter, simply referred to as a "received signal"), it is possible to generate reflected wave data, such as a temporal change or position information of a structure in the living body of the subject 2002. Images in respective modes of so-called A mode, B mode, M mode, and color Doppler are included in the reflected wave data. Measurement using an ultrasonic wave is repeatedly performed at predetermined periods. The measurement unit is referred to as a "frame".

By setting tracking point (a region of interest) in the received signal as a reference, the ultrasonic measurement apparatus 2010 can perform so-called "tracking" that is tracking the tracking point in time series between different frames and calculating the displacement. In the second embodiment, a front wall and a rear wall of the blood vessel 2004 are set as the tracking points, and the ultrasonic measurement apparatus 2010 has characteristics relating to the setting and starting control of the tracking points.

### Description of Principle of Second Embodiment

Principles of the setting of the tracking points and the starting control will be described.

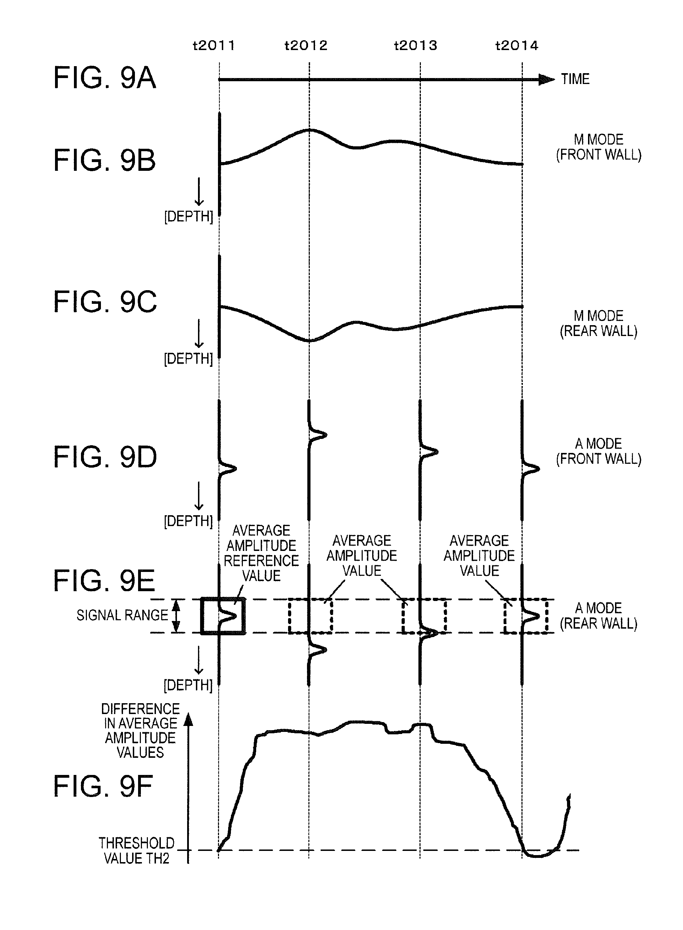

Figs. 9A to 9F are diagrams schematically showing the reflected wave data obtained by signal processing of the received data, in which Fig. 9A shows a time axis, Fig. 9B shows a schematic M mode image relating to the front wall, Fig. 9C shows a schematic M mode image relating to the rear wall, Fig. 9D shows a schematic A mode image relating to the front wall, and Fig. 9E shows a schematic A mode image relating to the rear wall. The A mode image can be referred to as the received signal as it is, and therefore, the A mode image will be described as the same meaning as the received signal, hereinafter. In the ultrasonic measurement, the reflected wave from the front wall and the rear wall of the blood vessel 2004 is detected strongly. The signal parts of the received signal shown in Figs. 9D and 9E are obtained by cutting out a circumferential part including the signal parts from the front wall and the rear wall, and are signal parts in a constant depth range.

The front wall and the rear wall of the blood vessel 2004 are displaced back and forth when seen from the ultrasonic probe 2016 (in depth direction) due to the pulsation. Accordingly, the part of the front wall and the part of the rear wall among the reflected wave signal are displaced as shown in Figs. 9B to 9E. Time points t2011 to t2014 are for one pulse. Herein, the front wall and the rear wall are displaced, but return to the same position after the one pulse. For example, in Fig. 9E, peaks of the rear walls are shown in substantially the same depth position in a signal waveform at the time point t2011 and in a signal waveform at the time point t2014. Needless to say, the peaks are shown in substantially the same position, after the integer number of pulses, not after the one pulse. In the second embodiment, the starting control of the tracking is performed using this.

First, a target desired to be tracked is designated and set from the received signal obtained at the first timing. It is possible to set a tracking target from the received signals obtained at the first timing as a so-called still image, and accordingly, it is possible to precisely set the tracking target by magnifying the received signal, for example. Fig. 9E shows an example in which the rear wall is set as a tracking target. The time point t2011 is the first timing. Among the received signals at the time point t2011, a depth range including the signal part of the rear wall is set as a signal range (depth range) desired to be the tracking target. This signal range is a region surrounded with a thick line. When the signal range is set, a signal component index value which is an index value showing the signal component in the signal range is calculated. In the second embodiment, as an example of the signal component index value, an average amplitude value obtained by averaging the amplitude of the signal is calculated. The signal component index value at the first timing is referred to as an index reference value, and the average amplitude value at the first timing is referred to as an average amplitude reference value. As the signal component index value, a total value of the amplitude in the signal range may be used, or a mean value of the amplitude in the signal range may be used, in addition to the average amplitude value.

When the setting of the signal range is completed, hereinafter, the acquired received signal is continuously monitored in real time. Specifically, the average amplitude value (an example of signal component index value) obtained by averaging the amplitude of the signal in the signal range among the received signals is calculated, and a second timing, at which a difference between the average amplitude value and the average amplitude reference value (hereinafter, this difference is referred to as a "difference in average amplitude values") satisfies the threshold value conditions is detected. That is, the second timing at which equivalent conditions in which the average amplitude value of the signal part in the signal range among the received signals corresponds to the average amplitude value at the first timing are satisfied, is continuously monitored. The tracking is started at this second timing.

Fig. 9F shows a change in the difference in the average amplitude values. A timing at which the difference in the average amplitude values is equal to or smaller than a threshold value is detected as the second timing. The time point t2014 is the second timing. As shown in Fig. 9E, at the time point t2014, the rear wall is positioned in the same depth position as the time point t2011 which is the first timing. Accordingly, a possibility of the part desired to be tracked and the part to actually be tracked being different from each other is extremely low. Since the tracking is started from the received signal acquired at the second timing (time point t2014), the tracking point (region of interest) is set from the received signal acquired at the second timing. Specifically, the tracking is started by using the signal part of the signal range as the tracking point (region of interest), among the received signal acquired at the second timing.

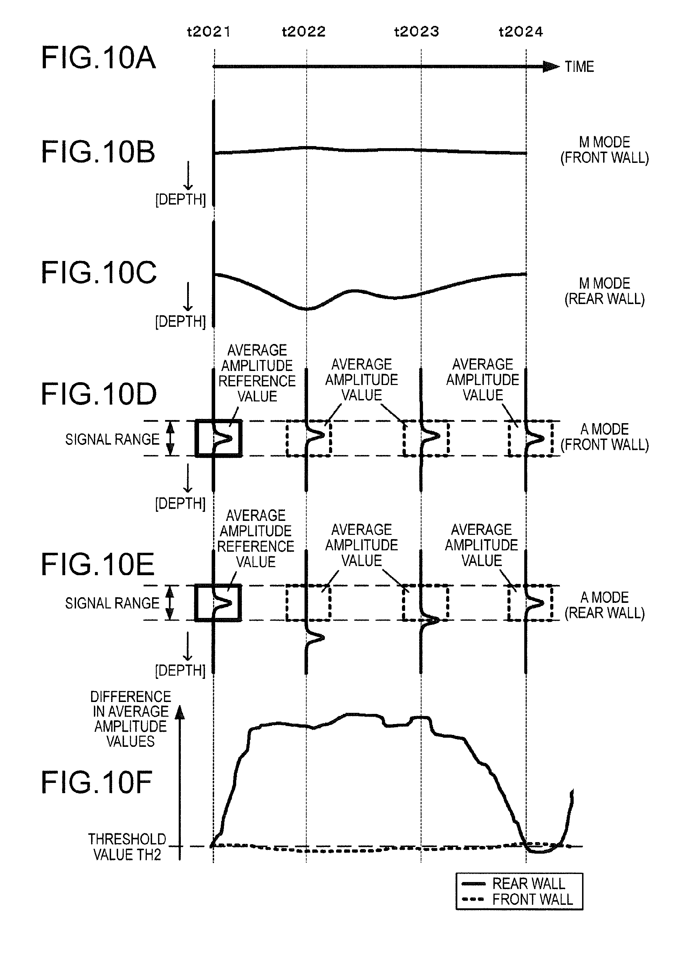

The tracking target may be only one of the front wall and the rear wall, but both walls are designated and set in the second embodiment. This is because a displacement amount of one of the front wall and the rear wall may be great, while a displacement amount of the other one is small. This phenomenon may occur due to a vascular state or vascular surrounding tissues and may occur depending on a blood vessel to be measured, a position to be measured, or a subject. Figs. 10A to 10F show an example of a case in which the displacement amount of one of the front wall and the rear wall is great and the displacement amount of the other one is small. Figs. 10A to 10F show a case in which the displacement amount of the front wall is small and the displacement amount of the rear wall is great. Figs. 10A to 10F are shown in the same manner as Figs. 9A to 9F.

In Figs. 10A to 10F, the depth positions of the front wall are substantially the same positions regardless of the pulsation. Accordingly, as shown in Fig. 10F, the difference in average amplitude values relating to the front wall is substantially the constant value, and the second timing at which the difference is equal to or greater than a threshold value TH2 may be before a time point t2024. By designating and setting both of the front wall and the rear wall as the tracking targets, it is possible to more accurately detect the second timing.

### Description of Functional Configuration of Second Embodiment

Next, the functional configuration for realizing the second embodiment will be described.

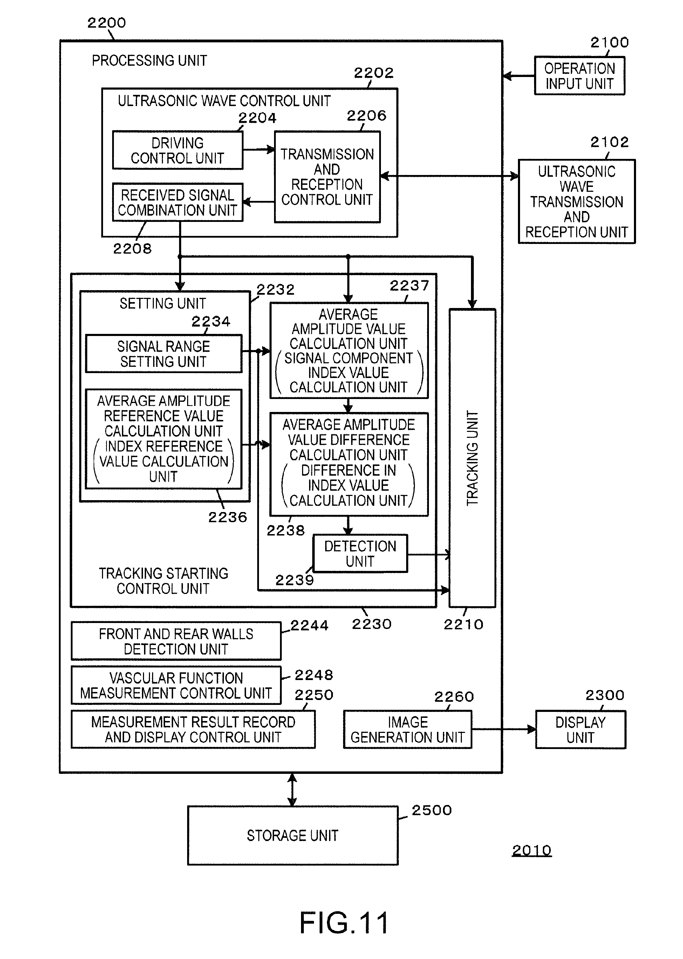

Fig. 11 is a block diagram showing an example of the functional configuration of the ultrasonic measurement apparatus 2010 in the second embodiment. The ultrasonic measurement apparatus 2010 includes an operation input unit 2100, an ultrasonic wave transmission and reception unit 2102, a processing unit 2200, a display unit 2300, and a storage unit 2500.

The operation input unit 2100 receives various kinds of operation input by the operator, and outputs an operation input signal corresponding to the operation input to the processing unit 2200. The operation input unit 2100 can be implemented by a button switch, a lever switch, a dial switch, a track pad, a mouse, or the like. In the example shown in Fig. 8, the touch panel 2012 or the keyboard 2014 corresponds to the operation input unit.

The ultrasonic wave transmission and reception unit 2102 transmits an ultrasonic wave with a pulse voltage output from the processing unit 2200. Then, the ultrasonic wave transmission and reception unit receives a reflected wave of the transmitted ultrasonic wave, converts the reflected wave into an electrical signal, and outputs the electrical signal to the processing unit 2200. The ultrasonic probe 2016 shown in Fig. 8 corresponds to the ultrasonic wave transmission and reception unit.

The processing unit 2200 is realized by a microprocessor, such as a CPU or a graphic processor unit (GPU), or an electronic component, such as an ASIC, field-programmable gate array (FPGA) or an IC memory, for example. In addition, the processing unit 2200 performs control of the input and output of data to each functional unit, and calculates biological information of the subject 2002 by performing various kinds of arithmetic processing based on a predetermined program or data, the operation input signal from the operation input unit 2100, the signal from the ultrasonic wave transmission and reception unit 2102, or the like. The processor 2030 and the control board 2031 shown in Fig. 8 correspond to the processing unit 2200.

The processing unit 2200 includes an ultrasonic wave control unit 2202, a tracking unit 2210, a tracking starting control unit 2230, a front and rear walls detection unit 2244, a vascular function measurement control unit 2248, a measurement result record and display control unit 2250, and an image generation unit 2260.

The ultrasonic wave control unit 2202 controls the transmission of an ultrasonic wave toward the blood vessel and the reception of a reflected wave. For example, the ultrasonic wave control unit includes a driving control unit 2204, a transmission and reception control unit 2206, and a received signal combination unit 2208, and performs overall control of ultrasonic measurement. The ultrasonic wave control unit 2202 can be realized by the well-known technologies.

The driving control unit 2204 controls the transmission timing of ultrasonic pulses from the ultrasonic probe 2016, and outputs a transmission control signal to the transmission and reception control unit 2206.

The transmission and reception control unit 2206 generates a pulse voltage according to the transmission control signal from the driving control unit 2204, and outputs the pulse voltage to the ultrasonic wave transmission and reception unit 2102. In this case, it is possible to adjust the output timing of the pulse voltage to each ultrasonic transducer by performing transmission delay processing. In addition, it is possible to perform the amplification or filtering of the signal output from the ultrasonic wave transmission and reception unit 2102 and to output the result to the received signal combination unit 2208.

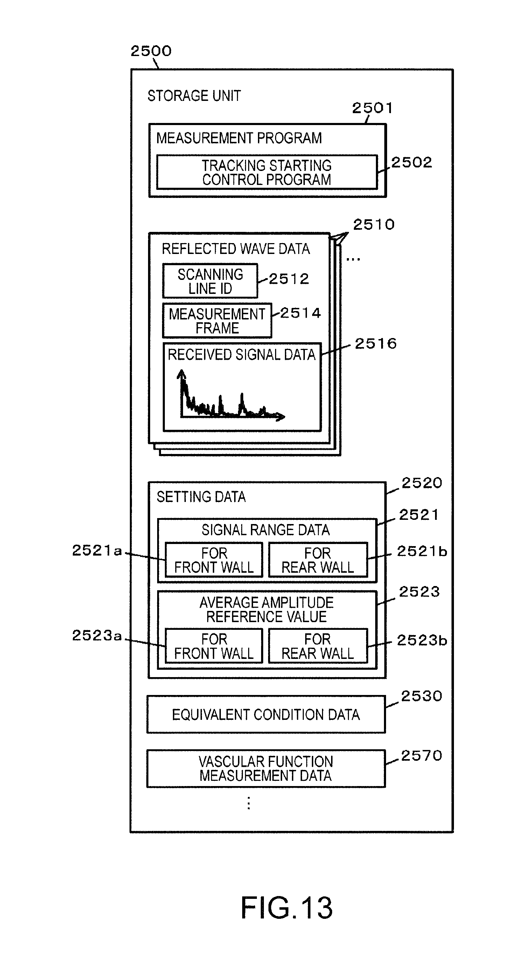

The received signal combination unit 2208 performs processing related to the so-called focus of a received signal by performing delay processing as necessary, thereby generating reflected wave data 2510 including received signal data 2516 (see Fig. 13). The reflected wave data 2510 is used in the tracking starting control unit 2230 and the tracking unit 2210.

The tracking starting control unit 2230 is a functional unit that performs starting control of the tracking described above, and includes a setting unit 2232, an average amplitude value calculation unit 2237 which is a calculation unit for the signal component index value, an average amplitude value difference calculation unit 2238 which is a calculation unit for the difference in the index values, and a detection unit 2239. In the second embodiment, the tracking starting control unit 2230 is realized as the software by reading out and executing a tracking starting control program 2502 (see Fig. 13) by the processing unit 2200, but the tracking starting control unit may be realized as the hardware by the electronic circuit such as an FPGA. The average amplitude value calculation unit 2237, the average amplitude value difference calculation unit 2238, and the detection unit 2239 may be configured with the electronic circuit such as an FPGA.

The setting unit 2232 is a functional unit that sets the tracking target, and includes a signal range setting unit 2234 , and an average amplitude reference value calculation unit 2236 which is a calculation unit for the index reference value. The signal range setting unit 2234 sets a position range (signal range) in a depth direction among the received signals at the first timing, according to the operation input of the operation input unit 2100. The signal range setting unit 2234 respectively sets signal ranges for the front wall and the rear wall and stores front wall signal range data 2521a and rear wall signal range data 2521b (collectively referred to as "signal range data 2521") in the storage unit 2500 (see Fig. 13).

The average amplitude reference value calculation unit 2236 calculates the index reference value which is the signal component index value in the signal range among the received signals at the first timing, that is, the average amplitude reference value. In the same manner as the signal range setting unit 2234, the average amplitude reference value calculation unit 2236 also respectively calculates the average amplitude reference value in the signal ranges for the front wall and the rear wall, and stores a front wall average amplitude reference value 2523a and a rear wall average amplitude reference value 2523b (collectively referred to as a "average amplitude reference value 2523") in the storage unit 2500.

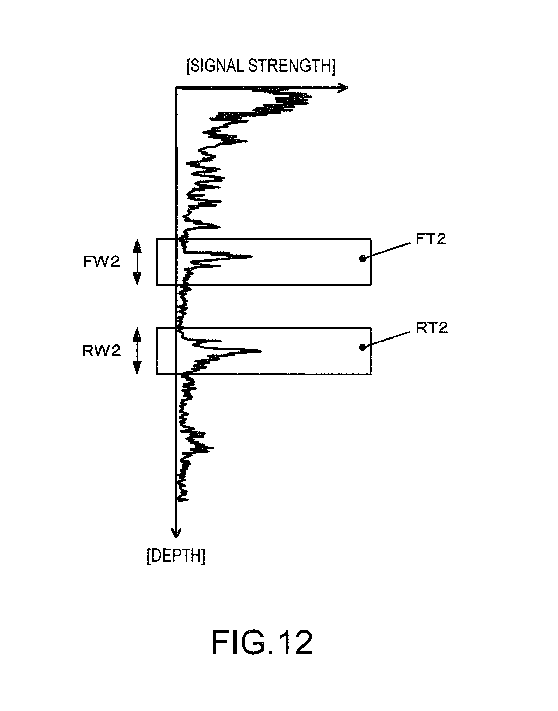

The setting of the signal range and the average amplitude reference value will be described in detail with reference to Fig. 12. Fig. 12 shows an example of a reflected wave signal obtained at a certain timing (first timing). The setting unit 2232, for example, displays the received signal on the display unit 2300 and sets the front wall signal range FW2 (2521a) and the rear wall signal range RW2 (2521b) according to the setting instruction operation from the operation input unit 2100. The signal ranges (front wall signal range FW2 and rear wall signal range RW2) are data for determining a range of the depth position. When setting the signal ranges, it is possible to accurately set the ranges by magnifying and displaying the received signal.

When the setting of the signal range is completed, the setting unit 2232 calculates the average amplitude value of the signal component in the front wall signal range FW2 and sets the average amplitude value as the front wall average amplitude reference value FT2 (2523a), and calculates the average amplitude value of the signal component in the rear wall signal range RW2 and sets the average amplitude value as the rear wall average amplitude reference value RT2 (2523b).

The description will be performed by returning to Fig. 11. The average amplitude value calculation unit 2237, the average amplitude value difference calculation unit 2238, and the detection unit 2239 function to detect the second timing.

The average amplitude value calculation unit 2237 calculates the average amplitude value of the signal component in the front wall signal range FW2 and the average amplitude value of the signal component in the rear wall signal range RW2 among the received signals output from the ultrasonic wave control unit 2202 on each occasion, and outputs the average amplitude values to the average amplitude value difference calculation unit 2238.

For the front wall and the rear wall, the average amplitude value difference calculation unit 2238 calculates the difference in the amplitude average values which is a difference between the average amplitude reference value and the average amplitude value calculated by the average amplitude value calculation unit 2237, and outputs the difference in the amplitude average values to the detection unit 2239.

The detection unit 2239 detects whether or not the difference in the amplitude average values satisfies the equivalent conditions, that is, the difference in the amplitude average values is equal to or smaller than the threshold value, thereby detecting the second timing. When the second timing is detected, the detection unit outputs a tracking starting instruction signal to the tracking unit 2210.

The tracking unit 2210 starts the tracking according to the tracking starting instruction signal from the detection unit 2239. The tracking points (regions of interest) are set in the signal parts in the signal ranges among the received signals at the time when the tracking starting instruction signal is input. Accordingly, the parts desired to be tracked are accurately set as the tracking points. For the tracking hereinafter, the well-known technologies can be used. For example, the functions of the "echo tracking" or the "phase difference tracking" are realized.

The front and rear walls detection unit 2244 detects vascular feature points from the received signal, as a previous step of the setting by the setting unit 2232. Specifically, two peaks which have signal strengths equal to or greater than a predetermined signal strength and in which the signal strength therebetween is equal to or smaller than a predetermined low strength indicating blood, are detected as the front wall and the rear wall. The detecting method of the front wall and the rear wall is not limited to this method, and the other methods may be used.

The vascular function measurement control unit 2248 determines the displacement of the blood vessel diameter based on the positions of the front wall and the rear wall of the blood vessel 2004 which are continuously measured by the tracking unit 2210, and performs the control relevant to predetermined vascular function measurement. For example, the operation control of executing processing of estimating a blood pressure from the blood vessel diameter using a stiffness parameter and measuring the blood pressure is performed.

The measurement result record and display control unit 2250 performs control for storing the measurement result of the vascular function in the storage unit 2500 and displaying the measurement result on the display unit 2300.

The image generation unit 2260 generates an image for displaying a measurement result or various operation screens required for ultrasonic measurement or biological information measurement, and outputs the image to the display unit 2300.

The display unit 2300 displays image data input from the image generation unit 2260. The touch panel 2012 shown in Fig. 8 corresponds to the display unit.

The storage unit 2500 is realized by a storage medium, such as an IC memory, a hard disk, or an optical disc, and stores various programs or various kinds of data, such as data in the operation process of the processing unit 2200. In Fig. 8, the storage medium 2033 mounted in the control board 2031 of the processor 2030 corresponds to the storage unit. In addition, the connection between the processing unit 2200 and the storage unit 2500 is not limited to a connection using an internal bus circuit in the apparatus, and may be realized by using a communication line, such as a local area network (LAN) or the Internet. In this case, the storage unit 2500 may be realized by using a separate external storage device from the ultrasonic measurement apparatus 2010.

In addition, as shown in Fig. 13, the storage unit 2500 stores a measurement program 2501, reflected wave data 2510, setting data 2520, equivalent condition data 2530, and vascular function measurement data 2570. Needless to say, frame identification information, various flags, counter values for time checking, and the like other than those described above can also be appropriately stored.

The processing unit 2200 realizes the functions of the ultrasonic wave control unit 2202, the tracking starting control unit 2230, the front and rear walls detection unit 2244, the vascular function measurement control unit 2248, the measurement result record and display control unit 2250, and the image generation unit 2260, by reading out and executing the measurement program 2501. The tracking starting control program 2502 for realizing the function of the tracking starting control unit 2230 is included in the measurement program 2501 as a subroutine program.

In addition, when realizing these functional units with hardware, such as an electronic circuit, a part of the program for realizing the function can be omitted.

The reflected wave data 2510 is reflected wave data obtained by ultrasonic measurement, and is generated for each frame by the ultrasonic wave control unit 2202. A piece of reflected wave data 2510, for example, includes a scanning line ID 2512, a measurement frame 2514, and received signal data (depth-signal strength data) 2516. Needless to say, data other than those described above can also be appropriately stored.

The setting data 2520 stores the signal range data 2521 which is data of the signal ranges set by the setting unit 2232, and the average amplitude reference value 2523, based on the received signal at the first timing.

The equivalent condition data 2530 is data for determining the threshold value conditions of the difference in the average amplitude values for the detection unit 2239 to detect the second timing, and the threshold values TH2 of Figs. 9A to 10F are determined, for example.

The vascular function measurement data 2570 stores the data in which the positions of the front wall and the rear wall of the blood vessel 2004 tracked by the tracking unit 2210 correspond to the time information at which the corresponding received signal is obtained, the data of the blood vessel diameter calculated from the positions of the front wall and the rear wall, or the data of the blood pressure calculated based on the blood vessel diameter.

### Description of Flow of Process of Second Embodiment

Next, the operation of the measurement process of the ultrasonic measurement apparatus 2010 will be described.

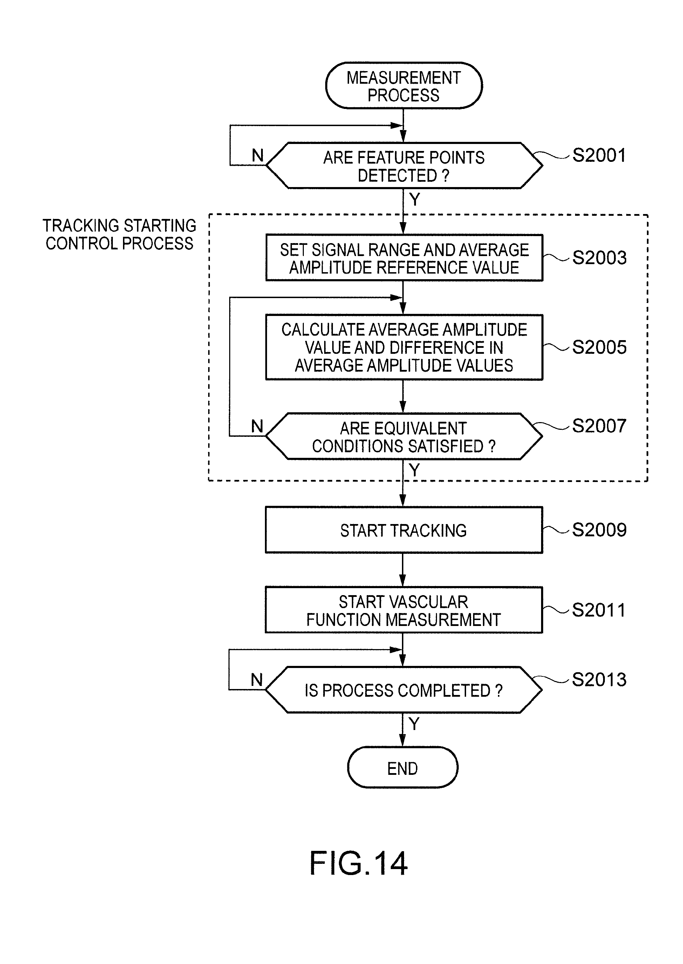

Fig. 14 is a flowchart for illustrating a flow of measurement process of the ultrasonic measurement apparatus 2010 in the second embodiment.

In this process, the processing unit 2200 starts a process of transmitting an ultrasonic beam for each ultrasonic transducer (scanning line) of the ultrasonic wave transmission and reception unit 2102, receiving the reflected wave, and storing the reflected wave data 2510 in the storage unit 2500 on each occasion.

First, the front and rear walls detection unit 2244 determines whether or not the feature points are included in the received signal, based on the received signal data 2516 obtained by the transmission and reception of the ultrasonic waves on each occasion, and therefore stands by until the feature points are detected (Step S2001: No). In the second embodiment, the feature points are peak indicating the front wall and the rear wall of the blood vessel 2004. When the feature points are detected (Step S2001: YES), the tracking starting control process is performed by the tracking starting control unit 2230 (Steps S2003 to S2007).

That is, the setting unit 2232 sets the signal range and the average amplitude reference value using the received signal obtained at the first timing (Step S2003). Then, the average amplitude value calculation unit 2237 calculates the average amplitude value in the signal range among the subsequent received signals on each occasion, and the average amplitude value difference calculation unit 2238 calculates the difference in the average amplitude values which is a difference between the average amplitude value and the average amplitude reference value set in Step S2003 (Step S2005). The detection unit 2239 determines whether or not the difference in the average amplitude values satisfies the equivalent conditions (threshold value conditions), that is, the second timing (Step S2007). The processes in Step S2005 to S2007 are repeated and the second timing is monitored, until the equivalent conditions are satisfied (Step S2007: NO).

When the equivalent conditions are satisfied (Step S2007: YES), the detection unit 2239 outputs the tracking starting instruction signal to the tracking unit 2210 when the second timing is detected, and the tracking unit 2210 starts the tracking (Step S2009). The vascular function measurement control unit 2248 starts the measurement of the vascular function (Step S2011).

After that, when the completion operation of the measurement process is performed (Step S2013: YES), the measurement process ends.

Hereinabove, according to the second embodiment, the signal range including the signal part for tracking the movement of the blood vessel walls of the blood vessel due to the pulsation, among the received signals obtained by receiving the reflected wave of the ultrasonic wave transmitted to the blood vessel 2004, is set. This setting is performed based on the received signals obtained at the first timing. Thus, the setting can be performed based on the received signal at a certain time, as a still image, and therefore it is possible to accurately set the desired tracking target.

When the signal range is set, the average amplitude value which is the signal component index value in the signal range at the first timing when performing the above setting is calculated as the average amplitude reference value.

After the setting of the tracking target, the second timing when the average amplitude value of the signal part corresponding to the set signal range satisfies the average amplitude reference value and the predetermined equivalent conditions, is detected. The tracking is started at the second timing. Accordingly, although there is a difference in time between the first timing and the second timing, the part desired to be tracked and the part to actually be tracked are not different from each other, regardless of the problems regarding the difference in time.

In the second embodiment, it is possible to appropriately add, omit, and change the constituent elements. For example, in the second embodiment, the both of the front wall and the rear wall of the blood vessel are the tracking targets in the above description, but only one thereof may be the tracking target.

In addition, the signal component index value is not limited to the average amplitude value, and an amplitude total value, or a mean value of the sampled amplitude may be used. The equivalent conditions are set to have the difference in the average amplitude values to be equal to or smaller than the threshold value, but the temporal conditions may further added thereto. That is, when the timing at which the difference in the average amplitude values is equal to or smaller than the threshold value is continuously detected and a state in which a difference in time between time intervals of the detection timing is equal to or smaller than a certain difference in times is continued for predetermined time, the equivalent conditions may be satisfied.

### Third Embodiment

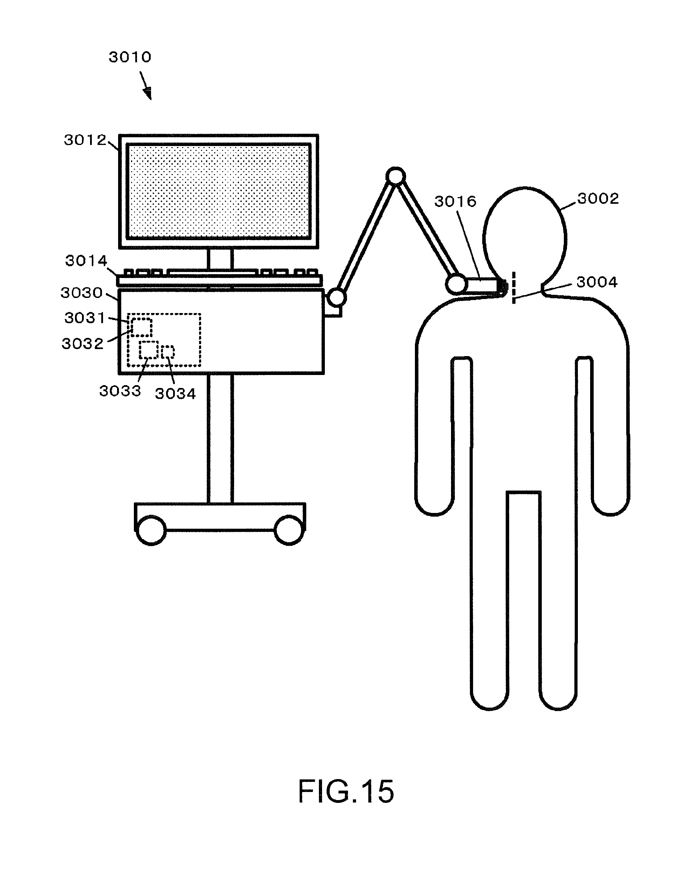

Fig. 15 is a diagram showing an example of the system configuration of an ultrasonic measurement apparatus 3010 according to a third embodiment. The ultrasonic measurement apparatus 3010 is an apparatus that measures biological information of a subject 3002 by measuring the reflected waves of ultrasonic waves. In the third embodiment, vascular function information such as blood pressure relating to a blood vessel 3004 which is an artery and an intima media thickness (IMT), is measured as a piece of biological information. In Fig. 15, a carotid artery is set as a blood vessel 3004 to be measured, but the other artery such as a radial artery may be set as the blood vessel 3004 to be measured.

The ultrasonic measurement apparatus 3010 includes a touch panel 3012 serving as a unit that displays a measurement result or operation information as an image and as an operation input unit, a keyboard 3014 used for operation input, an ultrasonic probe 3016, and a processor 3030. A control board 3031 is mounted in the processor 3030, and is connected to each unit of the apparatus, such as the touch panel 3012, the keyboard 3014, and the ultrasonic probe 3016, so that signal transmission and reception therebetween are possible.

Not only various integrated circuits, such as a central processing unit (CPU) 3032 and an application specific integrated circuit (ASIC), but also a storage medium 3033, such as an IC memory or a hard disk, and a communication IC 3034 for realizing data communication with an external device are mounted on the control board 3031. The processor 3030 realizes various functions according to the third embodiment such as a control operation for starting tracking of a vessel wall of the blood vessel 3004 according to the ultrasonic measurement and measurement of vascular function information for the blood vessel 3004 by executing a measurement program stored in the storage medium 3033 with the CPU 3032 or the like.

Specifically, by the control of the processor 3030, the ultrasonic measurement apparatus 3010 transmits and emits an ultrasonic beam from the ultrasonic probe 3016 to the subject 3002 and receives the reflected wave. Then, by performing signal processing on a received signal of the reflected wave (hereinafter, simply referred to as a "received signal"), it is possible to generate reflected wave data, such as a temporal change or position information of a structure in the living body of the subject 3002. Images in respective modes of so-called A mode, B mode, M mode, and color Doppler are included in the reflected wave data. Measurement using an ultrasonic wave is repeatedly performed at predetermined periods. The measurement unit is referred to as a "frame".

By setting tracking point (a region of interest) in the received signal as a reference, the ultrasonic measurement apparatus 3010 can perform so-called "tracking" that is tracking the tracking point in time series between different frames and calculating the displacement. In the third embodiment, a front wall and a rear wall of the blood vessel 3004 are set as the tracking points, and the ultrasonic measurement apparatus 3010 has characteristics relating to the setting and starting control of the tracking points.

### Description of Principle of Third Embodiment

Principles of the setting of the tracking points and the starting control will be described.

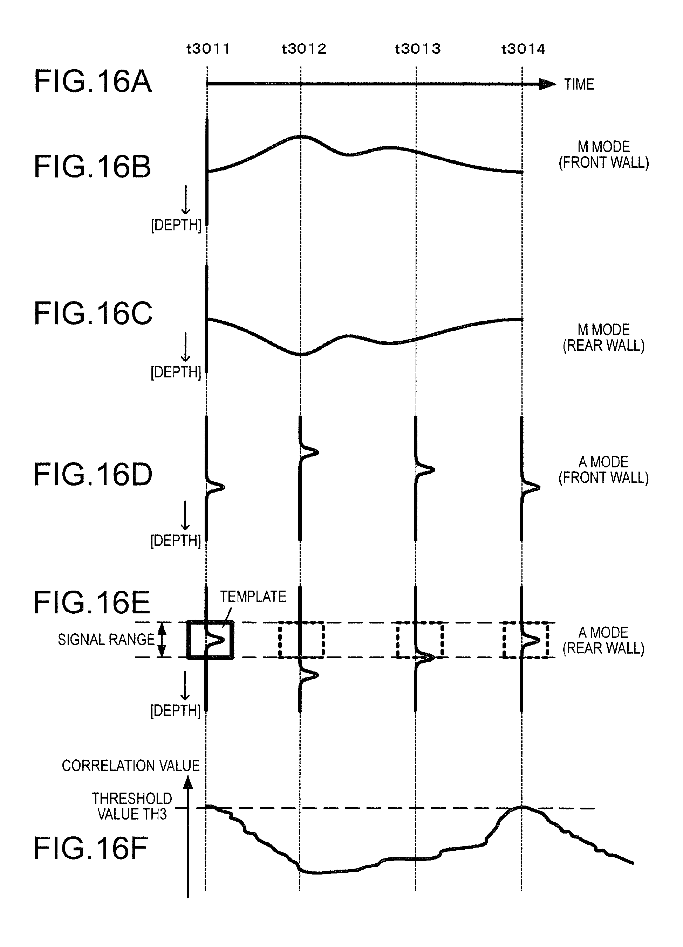

Figs. 16A to 16F are diagrams schematically showing the reflected wave data obtained by signal processing of the received signal, in which Fig. 16A shows a time axis, Fig. 16B shows a schematic M mode image relating to the front wall, Fig. 16C shows a schematic M mode image relating to the rear wall, Fig. 16D shows a schematic A mode image relating to the front wall, and Fig. 16E shows a schematic A mode image relating to the rear wall. The A mode image can be referred to as the received signal as it is, and therefore, the A mode image will be described as the same meaning as the received signal, hereinafter. In the ultrasonic measurement, the reflected wave from the front wall and the rear wall of the blood vessel 3004 is detected strongly. The signal parts of the received signal shown in Figs. 16D and 16E are obtained by cutting out a circumferential part including the signal parts from the front wall and the rear wall, and are signal parts in a constant depth range.

The front wall and the rear wall of the blood vessel 3004 are displaced back and forth when seen from the ultrasonic probe 3016 (in depth direction) due to the pulsation. Accordingly, the part of the front wall and the part of the rear wall among the reflected wave signal are displaced as shown in Figs. 16B to 16E. Time points t3011 to t3014 are for one pulse. Herein, the front wall and the rear wall are displaced, but return to the same position after the one pulse. For example, in Fig. 16E, peaks of the rear walls are shown in substantially the same depth position in a signal waveform at the time point t3011 and in a signal waveform at the time point t3014. Needless to say, the peaks are shown in substantially the same position, after the integer number of pulses, besides after the one pulse. In the third embodiment, the starting control of the tracking is performed using this.

First, a target desired to be tracked is designated and set from the received signal obtained at the first timing. It is possible to set a tracking target from the received signals obtained at the first timing as a so-called still image, and accordingly, it is possible to precisely set the tracking target by magnifying the received signal, for example. Fig. 16E shows an example in which the rear wall is set as a tracking target. The time point t3011 is the first timing. Among the received signals at the time point t3011, a depth range including the signal part of the rear wall is set as a signal range (depth range) desired to be the tracking target. This signal range is a region surrounded with a thick line. A signal waveform in the signal range is set as a template waveform.

When the setting of the signal range is completed, hereinafter, the acquired received signal is continuously monitored in real time. Specifically, a correlation operation of the signal waveform in the signal range among the received signals and the template waveform is repeatedly performed, and a second timing, at which a correlation value satisfies threshold value conditions is detected. That is, the second timing at which equivalent conditions in which the signal waveform in the signal range among the received signals corresponds to the template waveform are satisfied, is continuously monitored. The tracking is started at this second timing.

Fig. 16F shows a change in the correlation value. A timing at which the correlation value is equal to or greater than a threshold value is detected as the second timing. The time point t3014 is the second timing. As shown in Fig. 16E, at the time point t3014, the rear wall is positioned in the same depth position as the time point t3011 which is the first timing. Accordingly, the part desired to be tracked and the part to actually be tracked are not different from each other. Since the tracking is started from the received signal acquired at the second timing (time point t3014), the tracking point (region of interest) is set from the received signal acquired at the second timing. Specifically, the tracking is started by using the signal part of the signal range as the tracking point (region of interest), among the received signals acquired at the second timing.

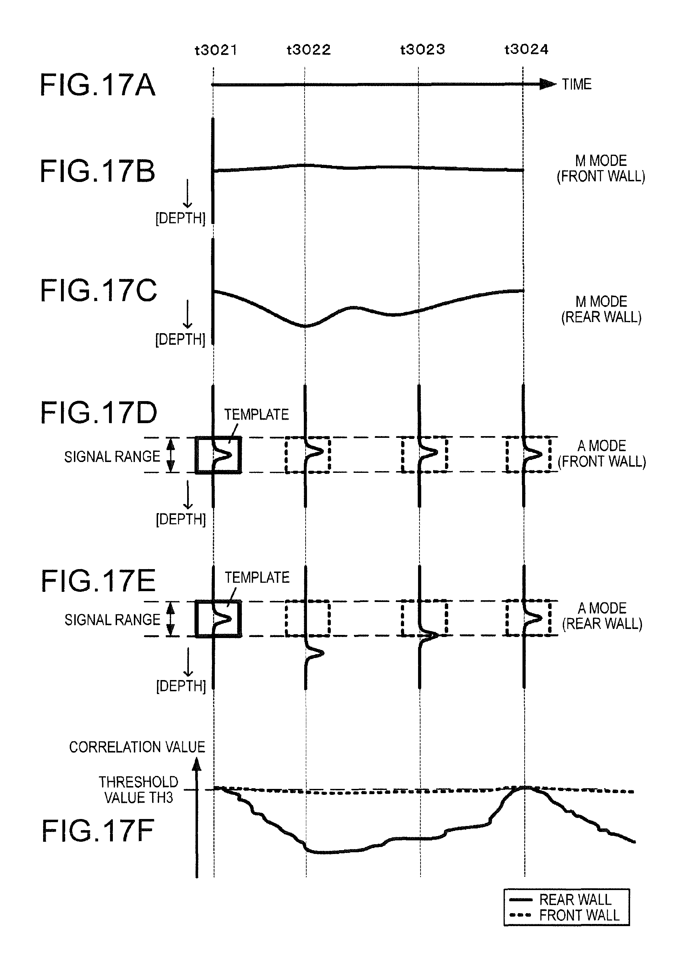

The tracking target may be only one of the front wall and the rear wall, but both walls are designated and set in the third embodiment. This is because a displacement amount of one of the front wall and the rear wall may be great, while a displacement amount of the other one is small. This phenomenon may occur due to a vascular state or vascular surrounding tissues and may occur depending on a blood vessel to be measured, a position to be measured, or a subject. Figs. 17A to 17F show an example of a case in which the displacement amount of one of the front wall and the rear wall is great and the displacement amount of the other one is small. Figs. 17A to 17F show a case in which the displacement amount of the front wall is small and the displacement amount of the rear wall is great. Figs. 17A to 17F are shown in the same manner as Figs. 16A to 16F.

In Figs. 17A to 17F, the depth positions of the front wall are substantially the same positions regardless of the pulsation. Accordingly, as shown in Fig. 17F, the correlation value relating to the front wall is substantially the constant value, and the second timing at which the correlation value is equal to or greater than a threshold value TH3 may be before a time point t3024. By designating and setting both of the front wall and the rear wall as the tracking targets, it is possible to more accurately detect the second timing.

Next, the setting of the first timing will be described.

The signal range and the template waveform may be set by setting the first timing as an arbitrary timing, but the blood vessel walls are displaced due to the pulsation. Accordingly, by determining a period in which the blood vessel walls are not displaced or the displacement amount is small and setting the period as the first timing, it is possible to more accurately detect the second timing.

The period in which the displacement of the blood vessel walls is small is during diastole, particularly telediastolic period. A method of determining the period and detecting the period as the first timing will be described with reference to the drawing.

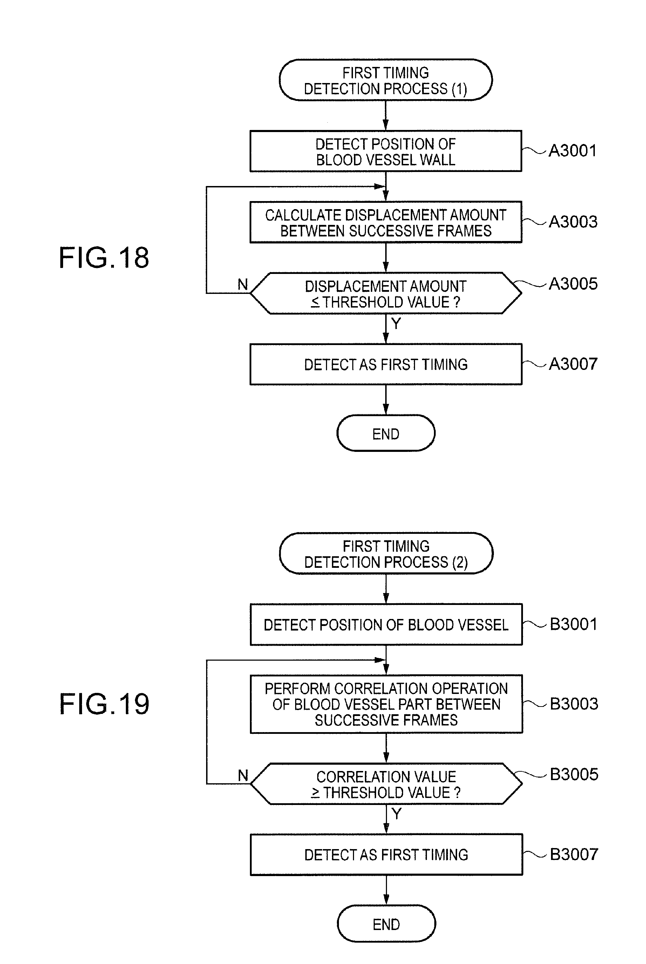

Fig. 18 is a diagram showing an example of a flow of a process of detecting the first timing and is a diagram showing a first process example. In the first process example, first, a position of the blood vessel wall is detected from the received signal of the reflected wave (Step A3001). That is, the position of the blood vessel wall is tracked in advance. The blood vessel wall to be detected may be one or both of the front wall and the rear wall. It is preferable to detect both thereof, in order to improve the accuracy. Then, a displacement amount of the blood vessel wall in successive preceding and succeeding frames is calculated (Step A3003). A timing when this displacement amount satisfies predetermined minute displacement conditions, that is, a timing when the displacement amount is equal to or smaller than a threshold value (StepA3005), is detected as the first timing (Step A3007). This method is a method using the fact that the displacement amount of the blood vessel wall is small during diastole. It is also possible to detect the telediastolic period depending on the threshold value in Step A3005.

Fig. 19 is a diagram showing a second process example. In the second process example, first, a position of the blood vessel is detected from the received signal of the reflected wave (Step B3001). That is, the position of the blood vessel is tracked in advance. Then, a correlation operation of signals of blood vessel parts among the received signals in successive preceding and succeeding frames is performed (Step B3003). A timing when this correlation value satisfies predetermined high correlation conditions, that is, a timing when the correlation value is equal to or greater than a threshold value (Step B3005), is detected as the first timing (Step B3007). This method is a method using the fact that the correlation of signals is high even with the received signals of different frames, because the displacement amount of the blood vessel is small during diastole. It is also possible to detect the telediastolic period depending on the threshold value in Step B3005.

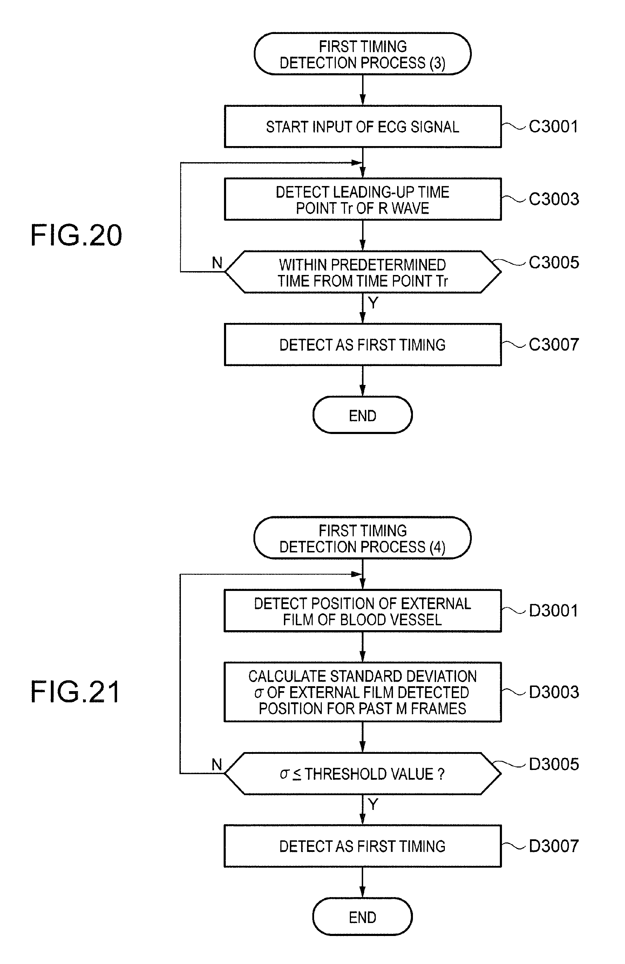

Fig. 20 is a diagram showing a third process example. In the third process example, the ultrasonic measurement apparatus 3010 inputs an electrocardiogram (ECG) signal of the subject 3002 from outside (Step C3001) and detects a leading-up time point Tr of an R wave appearing on the ECG signal (Step C3003). The predetermined time (for example, several ms) from the detected time point Tr, is detected as the first timing (Steps C3005 and C3007). This is a method using the fact that the diastole can be determined based on the R wave of the ECG signal. It is also possible to detect the telediastolic period depending on the setting of the determination period based on the time point Tr.

Fig. 21 is a diagram showing a fourth process example. In the fourth process example, first a position of a blood vessel external film is detected from the received signal of the reflected wave (Step D3001). That is, the position of the blood vessel external film is tracked in advance. Then, a standard deviation σ of the external film detection position for the past M frames from the preset is calculated (Step D3003). A timing when the standard deviation σ satisfies predetermined minute displacement conditions, that is, a timing when the standard deviation σ is equal to or smaller than a threshold value (Step D3005), is detected as the first timing (Step D3007). This method is a method using the fact that the variation of the position of the blood vessel external film is equal to or smaller than a certain value during diastole. It is also possible to detect the telediastolic period depending on the threshold value in Step D3005.

### Description of Functional Configuration of Third Embodiment

Next, the functional configuration for realizing the third embodiment will be described.

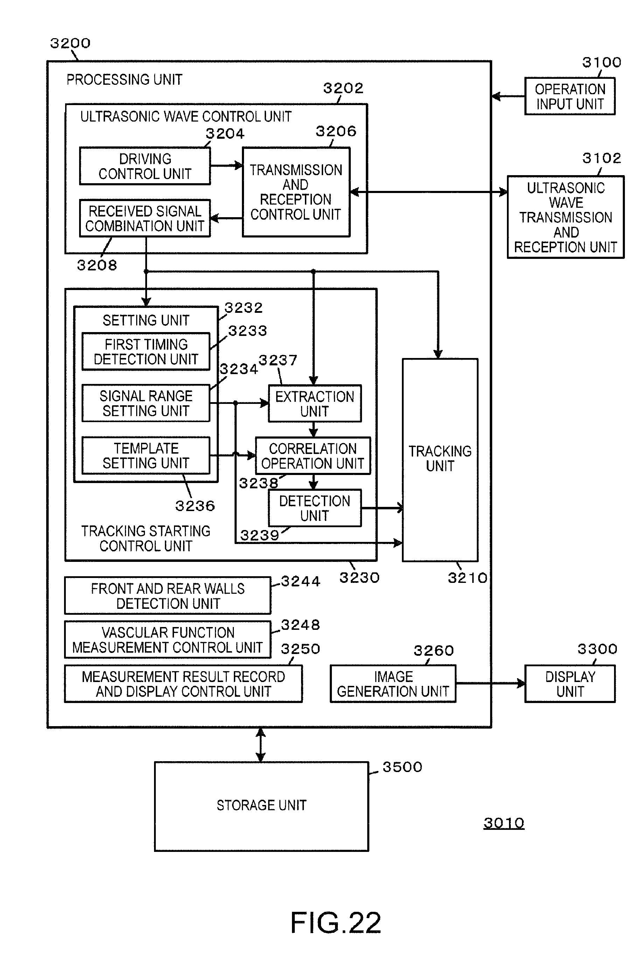

Fig. 22 is a block diagram showing an example of the functional configuration of the ultrasonic measurement apparatus 3010 in the third embodiment. The ultrasonic measurement apparatus 3010 includes an operation input unit 3100, an ultrasonic wave transmission and reception unit 3102, a processing unit 3200, a display unit 3300, and a storage unit 3500.

The operation input unit 3100 receives various kinds of operation input by the operator, and outputs an operation input signal corresponding to the operation input to the processing unit 3200. The operation input unit 3100 can be implemented by a button switch, a lever switch, a dial switch, a track pad, a mouse, or the like. In the example shown in Fig. 15, the touch panel 3012 or the keyboard 3014 corresponds to the operation input unit.

The ultrasonic wave transmission and reception unit 3102 transmits an ultrasonic wave with a pulse voltage output from the processing unit 3200. Then, the ultrasonic wave transmission and reception unit receives a reflected wave of the transmitted ultrasonic wave, converts the reflected wave into an electrical signal, and outputs the electrical signal to the processing unit 3200. The ultrasonic probe 3016 shown in Fig. 15 corresponds to the ultrasonic wave transmission and reception unit.

The processing unit 3200 is realized by a microprocessor, such as a CPU or a graphic processor unit (GPU), or an electronic component, such as an ASIC, field-programmable gate array (FPGA) or an IC memory, for example. In addition, the processing unit 3200 performs control of the input and output of data to each functional unit, and calculates biological information of the subject 3002 by performing various kinds of arithmetic processing based on a predetermined program or data, the operation input signal from the operation input unit 3100, the signal from the ultrasonic wave transmission and reception unit 3102, or the like. The processor 3030 and the control board 3031 shown in Fig. 15 correspond to the processing unit 3200.

The processing unit 3200 includes an ultrasonic wave control unit 3202, a tracking unit 3210, a tracking starting control unit 3230, a front and rear walls detection unit 3244, a vascular function measurement control unit 3248, a measurement result record and display control unit 3250, and an image generation unit 3260.

The ultrasonic wave control unit 3202 controls the transmission of an ultrasonic wave toward the blood vessel and the reception of a reflected wave. For example, the ultrasonic wave control unit includes a driving control unit 3204, a transmission and reception control unit 3206, and a received signal combination unit 3208, and performs overall control of ultrasonic measurement. The ultrasonic wave control unit 3202 can be realized by the well-known technologies.

The driving control unit 3204 controls the transmission timing of ultrasonic pulses from the ultrasonic probe 3016, and outputs a transmission control signal to the transmission and reception control unit 3206.

The transmission and reception control unit 3206 generates a pulse voltage according to the transmission control signal from the driving control unit 3204, and outputs the pulse voltage to the ultrasonic wave transmission and reception unit 3102. In this case, it is possible to adjust the output timing of the pulse voltage to each ultrasonic transducer by performing transmission delay processing. In addition, it is possible to perform the amplification or filtering of the signal output from the ultrasonic wave transmission and reception unit 3102 and to output the result to the received signal combination unit 3208.

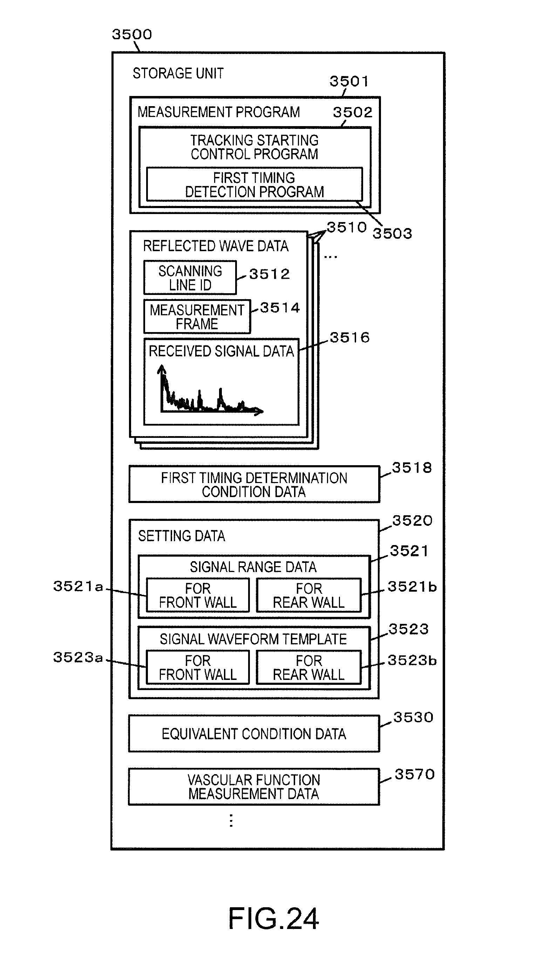

The received signal combination unit 3208 performs processing related to the so-called focus of a received signal by performing delay processing as necessary, thereby generating reflected wave data 3510 including received signal data 3516 (see Fig. 24). The reflected wave data 3510 is used in the tracking starting control unit 3230 and the tracking unit 3210.

The tracking starting control unit 3230 is a functional unit that performs starting control of the tracking described above, and includes a setting unit 3232, an extraction unit 3237, a correlation operation unit 3238, and a detection unit 3239. In the third embodiment, the tracking starting control unit 3230 is realized as the software by reading out and executing a tracking starting control program 3502 (see Fig. 24) by the processing unit 3200, but the tracking starting control unit may be realized as the hardware by the electronic circuit such as an FPGA. The extraction unit 3237, the correlation operation unit 3238, and the detection unit 3239 may be configured with the electronic circuit such as an FPGA.

The setting unit 3232 is a functional unit that sets the tracking target, and includes a first timing detection unit 3233 for detecting the first timing, a signal range setting unit 3234 that sets a position range (signal range) in a depth direction in the received signal according to the operation input of the operation input unit 3100, and a template setting unit 3236 that sets a signal waveform in the signal range in the received signal as a signal waveform template. The first timing detection unit 3233 is a functional unit that detects the first timing using any one of methods described with reference to Figs. 18 to 21. The threshold value conditions (predetermined minute displacement conditions) are stored as first determination condition data 3518 in the storage unit 3500, in order to determine the first timing.

The signal range setting unit 3234 respectively sets signal ranges for the front wall and the rear wall from the received signals at the first timing, and stores front wall signal range data 3521a and rear wall signal range data 3521b (collectively referred to as "signal range data 3521") in the storage unit 3500 (see Fig. 24). In the same manner as described above, the template setting unit 3236 also respectively sets signal waveform templates for the front wall and the rear wall and stores a front wall signal waveform template 3523a and a rear wall signal waveform template 3523b (collectively referred to as a "signal waveform template 3523") in the storage unit 3500.

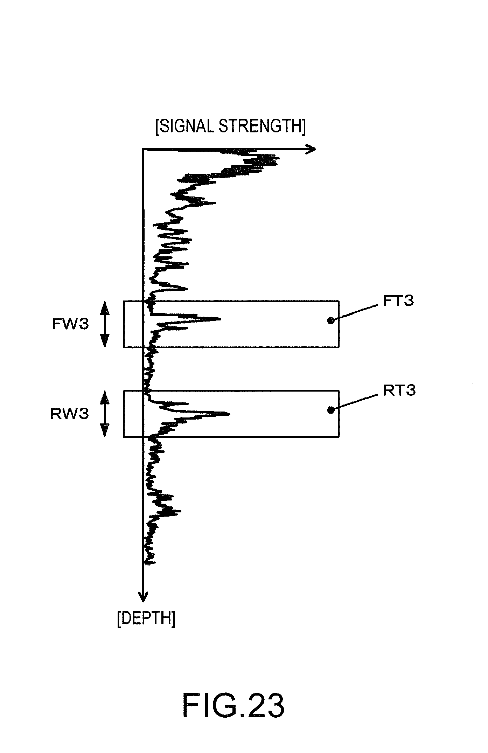

The setting of the signal range and the signal waveform template will be described in detail with reference to Fig. 23. Fig. 23 shows an example of a reflected wave signal obtained at the first timing. The setting unit 3232, for example, displays the received signal on the display unit 3300 and sets the front wall signal range FW3 (3521a) and the rear wall signal range RW3 (3521b) according to the setting instruction operation from the operation input unit 3100. The signal ranges (front wall signal range FW3 and rear wall signal range RW3) are data for determining a range of the depth position. When setting the signal ranges, it is possible to accurately set the ranges by magnifying and displaying the received signal.

When the setting of the signal range is completed, the setting unit 3232 cuts out a signal part in the front wall signal range FW3 and sets the signal part as the front wall signal waveform template FT3 (3523a), and cuts out a signal part in the rear wall signal range RW3 and sets the signal part as the rear wall signal waveform template RT3 (3523b).

The description will be performed by returning to Fig. 22. The extraction unit 3237, the correlation operation unit 3238, and the detection unit 3239 function to detect the second timing.

The extraction unit 3237 extracts the signal part in the front wall signal range FW3 and the signal part in the rear wall signal range RW3 among the received signals output from the ultrasonic wave control unit 3202 on each occasion, and outputs the signal parts to the correlation operation unit 3238.

For the front wall and the rear wall, the correlation operation unit 3238 performs the correlation operation of the signal waveform templates and the signal parts extracted by the extraction unit 3237, calculates a correlation value, and outputs the correlation value to the detection unit 3239. As the correlation operation, normalized cross-correlation or zero-mean normalized cross-correlation can be used, for example.

The detection unit 3239 detects whether or not the correlation value satisfies the equivalent conditions, that is, the correlation value is equal to or greater than the threshold value, thereby detecting the second timing. When the second timing is detected, the detection unit outputs a tracking starting instruction signal to the tracking unit 3210.

The tracking unit 3210 starts the tracking according to the tracking starting instruction signal from the detection unit 3239. The tracking points (regions of interest) are set in the signal parts in the predetermined signal ranges among the received signals at the time when the tracking starting instruction signal is input. Accordingly, the parts desired to be tracked are accurately set as the tracking points. For the tracking hereinafter, the well-known technologies can be used. For example, the functions of the "echo tracking" or the "phase difference tracking" are realized.

The front and rear walls detection unit 3244 detects vascular feature points from the received signal, as a previous step of the setting by the setting unit 3232. Specifically, two peaks which have signal strengths equal to or greater than a predetermined signal strength and in which the signal strength therebetween is equal to or smaller than a predetermined low strength indicating blood, are detected as the front wall and the rear wall. The detecting method of the front wall and the rear wall is not limited to this method, and the other methods may be used.

The vascular function measurement control unit 3248 determines the displacement of the blood vessel diameter based on the positions of the front wall and the rear wall of the blood vessel 3004 which are continuously measured by the tracking unit 3210, and performs the control relevant to predetermined vascular function measurement. For example, the operation control of executing processing of estimating a blood pressure from the blood vessel diameter using a stiffness parameter and measuring the blood pressure is performed, for example.

The measurement result record and display control unit 3250 performs control for storing the measurement result of the vascular function in the storage unit 3500 and displaying the measurement result on the display unit 3300.

The image generation unit 3260 generates an image for displaying a measurement result or various operation screens required for ultrasonic measurement or biological information measurement, and outputs the image to the display unit 3300.

The display unit 3300 displays image data input from the image generation unit 3260. The touch panel 3012 shown in Fig. 15 corresponds to the display unit.

The storage unit 3500 is realized by a storage medium, such as an IC memory, a hard disk, or an optical disc, and stores various programs or various kinds of data, such as data in the operation process of the processing unit 3200. In Fig. 15, the storage medium 3033 mounted in the control board 3031 of the processor 3030 corresponds to the storage unit. In addition, the connection between the processing unit 3200 and the storage unit 3500 is not limited to a connection using an internal bus circuit in the apparatus, and may be realized by using a communication line, such as a local area network (LAN) or the Internet. In this case, the storage unit 3500 may be realized by using a separate external storage device from the ultrasonic measurement apparatus 3010.

In addition, as shown in Fig. 24, the storage unit 3500 stores a measurement program 3501, reflected wave data 3510, first timing determination condition data 3518, setting data 3520, equivalent condition data 3530, and vascular function measurement data 3570. Needless to say, frame identification information, various flags, counter values for time checking, and the like other than those described above can also be appropriately stored.

The processing unit 3200 realizes the functions of the ultrasonic wave control unit 3202, the tracking starting control unit 3230, the front and rear walls detection unit 3244, the vascular function measurement control unit 3248, the measurement result record and display control unit 3250, and the image generation unit 3260, by reading out and executing the measurement program 3501. The tracking starting control program 3502 for realizing the function of the tracking starting control unit 3230 is included in the measurement program 3501 as a subroutine program. In addition, a first timing detection program 3503 for realizing the function of the first timing detection unit 3233 is included in the tracking starting control program 3502 as a subroutine program.

In addition, when realizing these functional units with hardware, such as an electronic circuit, a part of the program for realizing the function can be omitted.

The reflected wave data 3510 is reflected wave data obtained by ultrasonic measurement, and is generated for each frame by the ultrasonic wave control unit 3202. A piece of reflected wave data 3510, for example, includes a scanning line ID 3512, a measurement frame 3514, and received signal data (depth-signal strength data) 3516. Needless to say, data other than those described above can also be appropriately stored.

The first timing determination condition data 3518 is data for determining the conditions (threshold value described with reference to Figs. 18 to 21) for the first timing detection unit 3233 to determine the first timing.

The setting data 3520 stores the signal range data 3521 which is data of the signal ranges set by the setting unit 3232, and the signal waveform template 3523, based on the received signal at the first timing.

The equivalent condition data 3530 is data for determining the threshold conditions of the correlation value for the detection unit 3239 to detect the second timing, and the threshold value TH3 of Figs. 16A to 17F are determined, for example.

The vascular function measurement data 3570 stores the data in which the positions of the front wall and the rear wall of the blood vessel 3004 tracked by the tracking unit 3210 correspond to the time information at which the corresponding received signal is obtained, the data of the blood vessel diameter calculated from the positions of the front wall and the rear wall, or the data of the blood pressure calculated based on the blood vessel diameter.

### Description of Flow of Process of Third Embodiment

Next, the operation of the measurement process of the ultrasonic measurement apparatus 3010 will be described.

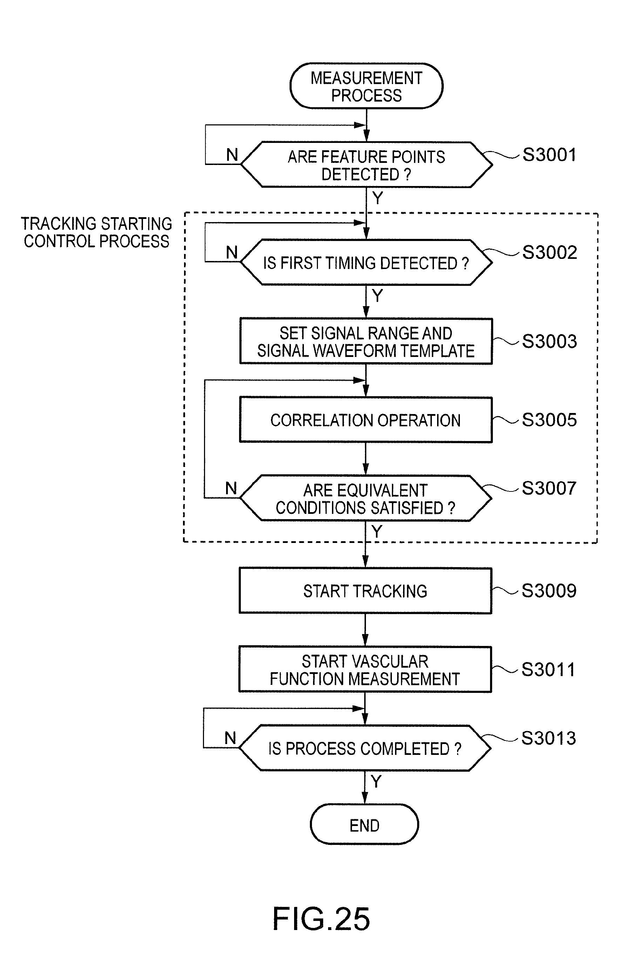

Fig. 25 is a flowchart for illustrating a flow of measurement process of the ultrasonic measurement apparatus 3010 in the third embodiment.

In this process, the processing unit 3200 starts a process of transmitting an ultrasonic beam for each ultrasonic transducer (scanning line) of the ultrasonic wave transmission and reception unit 3102, receiving the reflected wave, and storing the reflected wave data 3510 in the storage unit 3500 on each occasion.

First, the front and rear walls detection unit 3244 determines whether or not the feature points are included in the received signal, based on the received signal data 3516 obtained by the transmission and reception of the ultrasonic waves on each occasion, and therefore stands by until the feature points are detected (Step S3001: No). In the third embodiment, the feature points are peak indicating the front wall and the rear wall of the blood vessel 3004. When the feature points are detected (Step S3001: YES), the tracking starting control process is performed by the tracking starting control unit 3230 (Steps S3002 to S3007).

That is, first, the first timing detection unit 3233 detects the first timing (Step S3002). For the detecting method of the first timing, any methods described with reference to Figs. 18 to 21 may be used. When the first timing can be detected (Step S3002: YES), the setting unit 3232 sets the signal range and the signal waveform template using the received signal at the first timing (Step S3003). Then, the extraction unit 3237 extracts the signal part in the signal range among the subsequent received signals on each occasion, and the correlation operation unit 3238 determines whether or not the signal part corresponds to the signal waveform template using the equivalent condition data 3530. Specifically, the correlation operation unit 3238 performs the correlation operation of the signal part and the signal waveform template (Step S3005) and determines whether or not the correlation value satisfies the equivalent conditions (threshold value conditions), that is, the second timing (Step S3007). The processes in Step S3005 to S3007 are repeated and the second timing is monitored, until the equivalent conditions are satisfied (Step S3007: NO).

When the equivalent conditions are satisfied (Step S3007: YES), the detection unit 3239 outputs the tracking starting instruction signal to the tracking unit 3210 when the second timing is detected, and the tracking unit 3210 starts the tracking (Step S3009). The vascular function measurement control unit 3248 starts the measurement of the vascular function (Step S3011).

After that, when the completion operation of the measurement process is performed (Step S3013: YES), the measurement process ends.

Hereinabove, according to the third embodiment, the signal range including the signal part for tracking the movement of the blood vessel walls of the blood vessel due to the pulsation, and the signal waveform template in the signal range, among the received signals obtained by receiving the reflected wave of the ultrasonic wave transmitted to the blood vessel 3004, are set. This setting is performed based on the received signals obtained at the first timing. Thus, the setting can be performed based on the received signal at a certain time, as a still image, and therefore it is possible to accurately set the desired tracking target.

After the setting of the tracking target, the second timing when the signal part corresponding to the set signal range and the set signal waveform template among the received signals satisfy the predetermined equivalent conditions, is detected. The tracking is started at the second timing. In addition, the first timing is during diastole when the position change of the blood vessel wall is slight. Accordingly, the second timing is also during diastole. Accordingly, although there is a difference in time between the first timing and the second timing, a possibility of the part desired to be tracked and the part to actually be tracked being different from each other is extremely low, regardless of the problems regarding the difference in time.

In the third embodiment, it is possible to appropriately add, omit, and change the constituent elements. For example, in the third embodiment, the both of the front wall and the rear wall of the blood vessel are the tracking targets in the above description, but only one thereof may be the tracking target.

In addition, the correlation operation is performed in the above description, but instead of the similarity such as the correlation value, dissimilarity such as sum of squared difference (SSD) or sum of absolute difference (SAD) may be used. However, when using SSD or SAD, as the value decreases the similarity increases, and therefore the equivalent conditions (threshold value condition) are set to have the value equal to or smaller than the threshold value.

### Fourth Embodiment

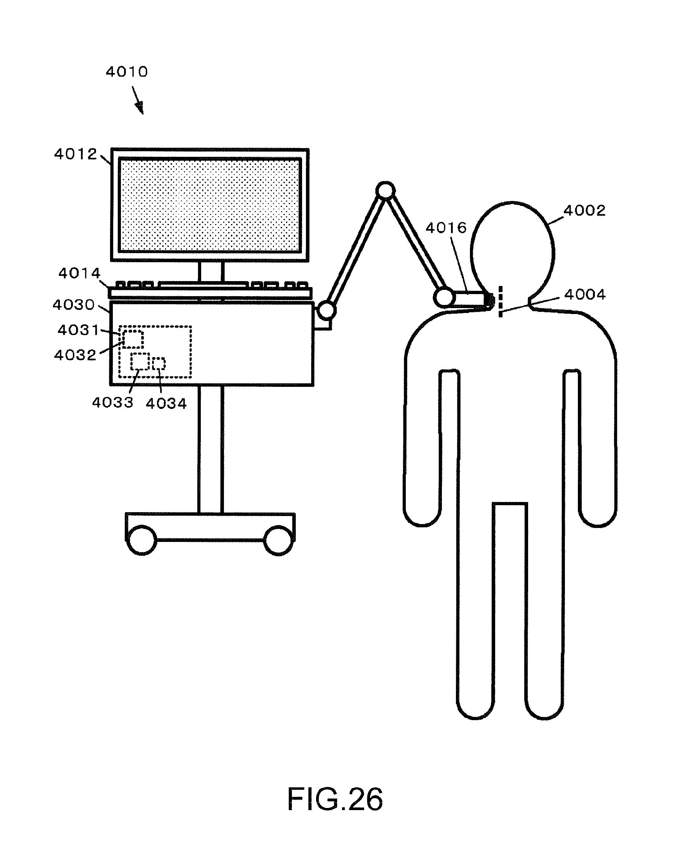

Fig. 26 is a diagram showing an example of the system configuration of an ultrasonic measurement apparatus 4010 according to a fourth embodiment. The ultrasonic measurement apparatus 4010 is an apparatus that measures biological information of a subject 4002 by measuring the reflected waves of ultrasonic waves. In the fourth embodiment, vascular function information such as blood pressure relating to a blood vessel 4004 which is an artery and an intima media thickness (IMT), is measured as a piece of biological information. In Fig. 26, a carotid artery is set as a blood vessel 4004 to be measured, but the other artery such as a radial artery may be set as the blood vessel 4004 to be measured.

The ultrasonic measurement apparatus 4010 includes a touch panel 4012 serving as a unit that displays a measurement result or operation information as an image and as an operation input unit, a keyboard 4014 used for operation input, an ultrasonic probe 4016, and a processor 4030. A control board 4031 is mounted in the processor 4030, and is connected to each unit of the apparatus, such as the touch panel 4012, the keyboard 4014, and the ultrasonic probe 4016, so that signal transmission and reception therebetween are possible.

Not only various integrated circuits, such as a central processing unit (CPU) 4032 and an application specific integrated circuit (ASIC), but also a storage medium 4033, such as an IC memory or a hard disk, and a communication IC 4034 for realizing data communication with an external device are mounted on the control board 4031. The processor 4030 realizes various functions according to the fourth embodiment such as a control operation for starting tracking of a vessel wall of the blood vessel 4004 according to the ultrasonic measurement and measurement of vascular function information for the blood vessel 4004 by executing a measurement program stored in the storage medium 4033 with the CPU 4032 or the like.

Specifically, by the control of the processor 4030, the ultrasonic measurement apparatus 4010 transmits and emits an ultrasonic beam from the ultrasonic probe 4016 to the subject 4002 and receives the reflected wave. Then, by performing signal processing on a received signal of the reflected wave (hereinafter, simply referred to as a "received signal"), it is possible to generate reflected wave data, such as a temporal change or position information of a structure in the living body of the subject 4002. Images in respective modes of so-called A mode, Bmode, Mmode, and color Doppler are included in the reflected wave data. Measurement using an ultrasonic wave is repeatedly performed at predetermined periods. The measurement unit is referred to as a "frame".

The ultrasonic measurement apparatus 4010 sets tracking point (a region of interest) in the received signal of the frame when starting the measurement. In the subsequent frames, the ultrasonic measurement apparatus can perform so-called "tracking" that is tracking the tracking point from the received signal of the frames and calculating the displacement. In the fourth embodiment, the blood vessel walls of the blood vessel 4004 are set as the tracking points, and the ultrasonic measurement apparatus 4010 has characteristics relating to the setting and starting control of the tracking points. In the fourth embodiment, the description will be performed by focusing on the rear wall as the blood vessel wall of the tracking target, but the front wall is also set as the tracking target in the same manner.

### Description of Principle of Fourth Embodiment

Principles of the setting of the tracking points and the starting control of the tracking will be described.

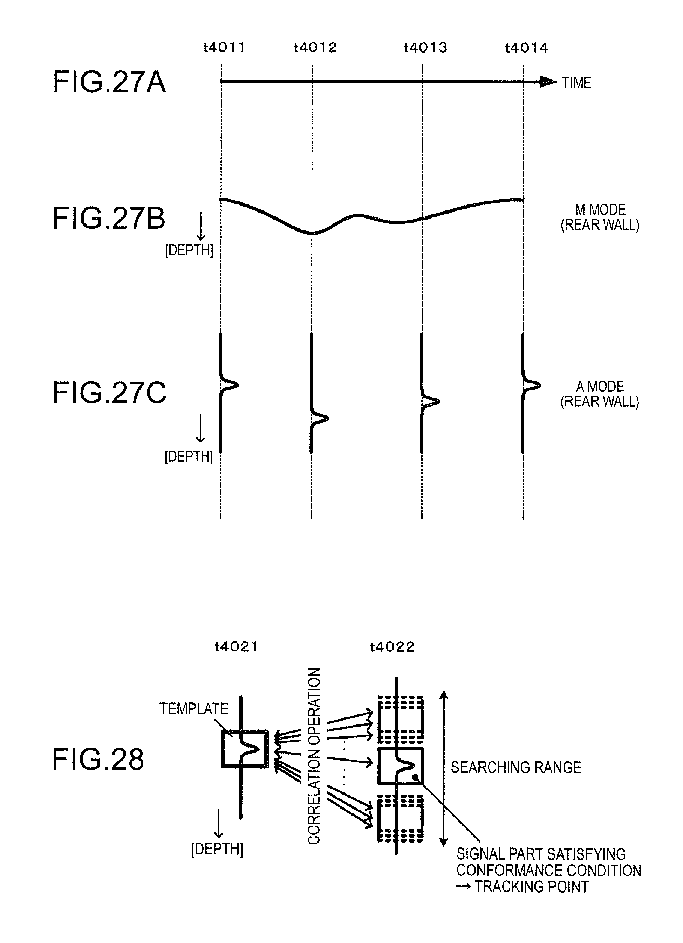

Figs. 27A to 27C are diagrams schematically showing the reflected wave data obtained by signal processing of the received signal, in which Fig. 27A shows a time axis, Fig. 27B shows a schematic M mode image relating to the rear wall, and Fig. 27C shows a schematic A mode image relating to the rear wall. The A mode image can be referred to as the received signal as it is, and therefore, the A mode image will be described as the same meaning as the received signal, hereinafter. In the ultrasonic measurement, the reflected wave from the front wall and the rear wall of the blood vessel 4004 is detected strongly. The signal parts of the received signal shown in Fig. 27C are obtained by cutting out a circumferential part including the signal parts from the rear wall, and are signal part in a constant depth range.

The front wall and the rear wall of the blood vessel 4004 are displaced back and forth when seen from the ultrasonic probe 4016 (in depth direction) due to the pulsation. For example, the parts of the rear wall are displaced as shown in Figs. 27B and 27C. Time points t4011 to t4014 are for one pulse. The front wall and the rear wall are displaced, but return to the same position after the one pulse. For example, in Fig. 27C, peaks of the rear walls are shown in substantially the same depth position in a signal waveform at the time point t4011 and in a signal waveform at the time point t4014. That is, among the received signals, the range in which the signal parts of the blood vessel wall are displaced is within a certain range.

Fig. 28 is a diagram for illustrating initial setting of the tracking point and shows the schematic A mode image relating to the rear wall. First, a timing point t4021 shown at the left of Fig. 28 is the first timing. A target desired to be tracked (signal waveform part of blood vessel wall) is designated and set from the received signal obtained at the first timing. It is possible to set a tracking target from the received signals obtained at the first timing as a so-called still image, and accordingly, it is possible to precisely set the tracking target by magnifying the received signal, for example. The part of the signal waveform of the set tracking target is set as the signal waveform template.

Next, when an instruction to start the tracking and to start the measurement of blood vessel diameter function information is input, the signal part conformable to the signal waveform template is searched from the received signals at the timing when the instruction is input (second timing), the tracking point is initially set in the position of the signal part, and the tracking is started. A time point t4022 shown at the right of Fig. 28 is the second timing.

The signal part conformable to the signal waveform template is searched as follows. That is, the signal part having the same width as the width (size of the range in the depth direction) of the signal range of the signal waveform template is cut out from the received signals at the second timing (time point t4022) and is selected as an operation target signal part. The correlation operation of the operation target signal part and the signal waveform template is performed. The selection of the operation target signal part and the correlation operation are performed by changing the operation target signal part in a predetermined searching range among the received signals at the second timing (time point t4022). For the searching range, a range assumed as a maximum displacement range of the rear wall is determined based on the depth position of the signal waveform template.

Then, among the correlation values for each selected position of the obtained operation target signal part, the tracking point is initially set in the selected position satisfying the predetermined conformance conditions. As the conformance conditions, the selected position having the highest correlation value may be used, or the selected position having the highest correlation value and having the correlation value equal to or greater than a predetermined value may be used, for example. When there is no selected position satisfying the conformance conditions, the position of the rear wall at the second timing may be beyond the searching range, and accordingly the selection of the operation target signal part and the correlation operation may be performed again by expanding the searching range.

Magnitude of the signal to be subjected to the correlation operation is a width (size of the range in the depth direction) of the signal waveform template, and the selection range of the operation target signal part is limited as the searching range. Accordingly, it is possible to rapidly search the operation target signal part satisfying the conformance conditions, and it is possible to execute the process in the frame interval time, even when the frame rate is, for example, 1 kHz. Therefore, the initial setting of the tracking point can be completed until the frame subsequent to the frame at the second timing when the measurement of the blood vessel diameter function information is started reaches. Thus, the tracking with the improved accuracy of the coincidence of the desired part to be tracked (part of the signal waveform template) and the part to actually be tracked (part set as the tracking point) is realized.

### Description of Functional Configuration of Fourth Embodiment

Next, the functional configuration for realizing the fourth embodiment will be described.

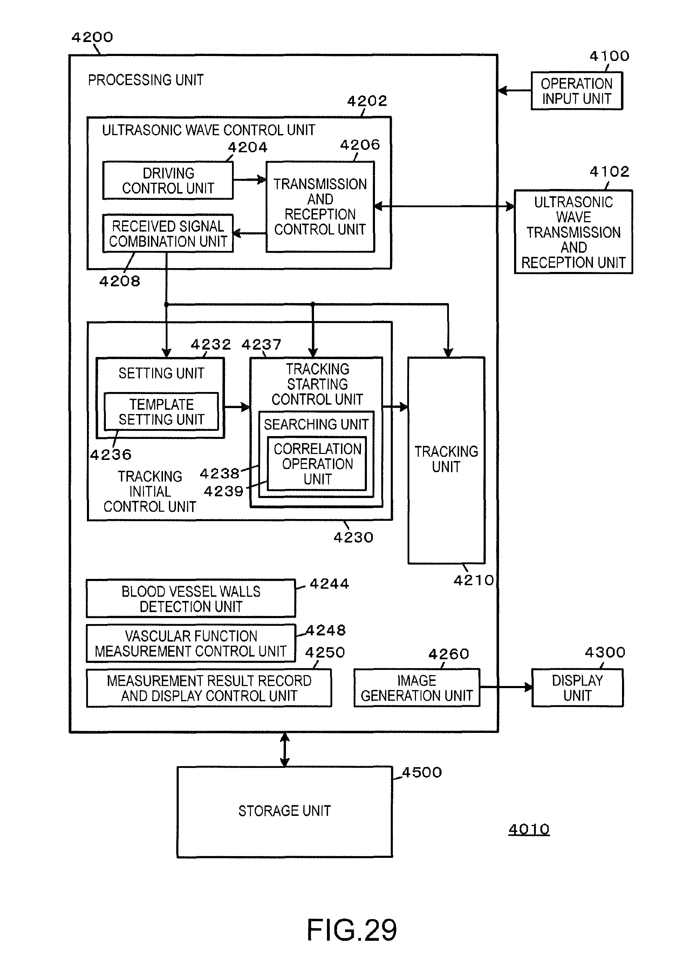

Fig. 29 is a block diagram showing an example of the functional configuration of the ultrasonic measurement apparatus 4010 in the fourth embodiment. The ultrasonic measurement apparatus 4010 includes an operation input unit 4100, an ultrasonic wave transmission and reception unit 4102, a processing unit 4200, a display unit 4300, and a storage unit 4500.

The operation input unit 4100 receives various kinds of operation input by the operator, and outputs an operation input signal corresponding to the operation input to the processing unit 4200. The operation input unit 4100 can be implemented by a button switch, a lever switch, a dial switch, a track pad, a mouse, or the like. In the example shown in Fig. 26, the touch panel 4012 or the keyboard 4014 corresponds to the operation input unit.

The ultrasonic wave transmission and reception unit 4102 transmits an ultrasonic wave with a pulse voltage output from the processing unit 4200. Then, the ultrasonic wave transmission and reception unit receives a reflected wave of the transmitted ultrasonic wave, converts the reflected wave into an electrical signal, and outputs the electrical signal to the processing unit 4200. The ultrasonic probe 4016 shown in Fig. 26 corresponds to the ultrasonic wave transmission and reception unit.

The processing unit 4200 is realized by a microprocessor, such as a CPU or a graphic processor unit (GPU), or an electronic component, such as an ASIC, field-programmable gate array (FPGA) or an IC memory, for example. In addition, the processing unit 4200 performs control of the input and output of data to each functional unit, and calculates biological information of the subject 4002 by performing various kinds of arithmetic processing based on a predetermined program or data, the operation input signal from the operation input unit 4100, the signal from the ultrasonic wave transmission and reception unit 4102, or the like. The processor 4030 and the control board 4031 shown in Fig. 26 correspond to the processing unit 4200.

The processing unit 4200 includes an ultrasonic wave control unit 4202, a tracking unit 4210, a tracking initial control unit 4230, a blood vessel walls detection unit 4244, a vascular function measurement control unit 4248, a measurement result record and display control unit 4250, and an image generation unit 4260.

The ultrasonic wave control unit 4202 controls the transmission of an ultrasonic wave toward the blood vessel and the reception of a reflected wave. For example, the ultrasonic wave control unit includes a driving control unit 4204, a transmission and reception control unit 4206, and a received signal combination unit 4208, and performs overall control of ultrasonic measurement.

The driving control unit 4204 controls the transmission timing of ultrasonic pulses from the ultrasonic probe 4016, and outputs a transmission control signal to the transmission and reception control unit 4206.

The transmission and reception control unit 4206 generates a pulse voltage according to the transmission control signal from the driving control unit 4204, and outputs the pulse voltage to the ultrasonic wave transmission and reception unit 4102. In this case, it is possible to adjust the output timing of the pulse voltage to each ultrasonic transducer by performing transmission delay processing. In addition, it is possible to perform the amplification or filtering of the signal output from the ultrasonic wave transmission and reception unit 4102 and to output the result to the received signal combination unit 4208.

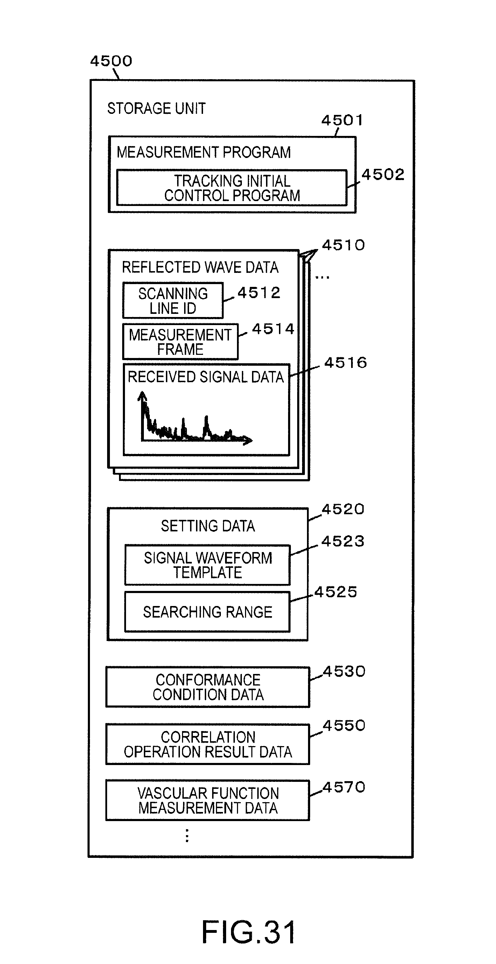

The received signal combination unit 4208 performs processing related to the so-called focus of a received signal by performing delay processing as necessary, thereby generating reflected wave data 4510 including received signal data 4516 (see Fig. 31). The reflected wave data 4510 is used in the tracking initial control unit 4230 and the tracking unit 4210.

The tracking initial control unit 4230 includes a template setting unit 4236 and a tracking starting control unit 4237. In the fourth embodiment, the tracking initial control unit 4230 is realized as the software by reading out and executing a tracking initial control program 4502 (see Fig. 31) by the processing unit 4200, but the tracking initial control unit may be realized as the hardware by the electronic circuit such as an FPGA. The template setting unit 4236 or the tracking starting control unit 4237 may be configured with the electronic circuit such as an FPGA, separately.

As described above, the template setting unit 4236 sets the signal part showing the waveforms of the tracking target (waveforms of the front wall and the rear wall of the blood vessel wall) as the signal waveform template. It is possible to precisely set the signal waveform template from the received signals obtained at the first timing by magnifying the received signals a so-called still image. The setting of the signal waveform template can be manually performed by an operator, but the setting may be automatically executed by a program of extracting the signal part of the blood vessel wall.

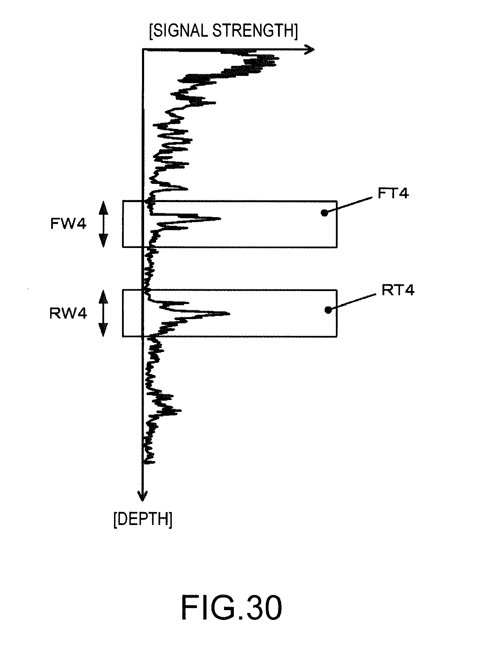

The setting of the signal waveform template will be described in detail with reference to Fig. 30. Fig. 30 is a diagram showing an example of the reflected wave signal obtained at the first timing. The template setting unit 4236, for example, displays the received signal on the display unit 4300 and specifies a front wall signal range FW4 and a rear wall signal range RW4 according to the setting instruction operation from the operation input unit 4100. The signal ranges are data for determining a range of the depth position. When specifying the signal ranges, it is possible to accurately set the ranges by magnifying and displaying the received signal.

The template setting unit 4236 sets the signal waveform in the specified signal range as the signal waveform template 4523 (see Fig. 31). The signal waveform in the front wall signal range FW4 is set as a front wall signal waveform template FT4, and the rear wall signal range RW4 is set as a rear wall signal waveform template RT4.

The tracking starting control unit 4237 is a functional unit initially sets the tracking points using the set signal waveform templates and controls to start the tracking, when an instruction to start the measurement is input to the operation input unit 4100, and includes a searching unit 4238 including a correlation operation unit 4239. The basic operation principle is as described above with reference to Fig. 28. That is to say, the operation target signal part is selected from the received signals at the second timing when the measurement is started and the correlation operation of the operation target signal part and the signal waveform template is performed. This is performed by changing the operation target signal part in a predetermined searching range among the received signals at the second timing, and accordingly, the searching unit 4238 searches the selected position satisfying the predetermined conformance conditions among the correlation values for each selected position of the operation target signal part. The tracking point is initially set in the selected position (signal part) satisfying the predetermined conformance conditions. When the setting of the tracking point is completed, the tracking starting control unit 4237 outputs the initially set information of the tracking points to the tracking unit 4210 and starts the tracking.

The tracking unit 4210 starts the tracking according to the tracking starting instruction signal from the tracking starting control unit 4237. The tracking points (regions of interest) are set in the received signal at the second timing, based on the initially set information from the tracking starting control unit 4237. Accordingly, the parts desired to be tracked are accurately set as the tracking points. For the tracking hereinafter, the well-known technologies can be used. For example, the functions of the "echo tracking" or the "phase difference tracking" are realized.

The blood vessel walls detection unit 4244 detects vascular feature points from the received signal, as a previous step of the setting by the template setting unit 4236. Specifically, two peaks which have signal strengths equal to or greater than a predetermined signal strength and in which the signal strength therebetween is equal to or smaller than a predetermined low strength indicating blood, are detected as the front wall and the rear wall. The detecting method of the front wall and the rear wall is not limited to this method, and the other methods may be used.

The vascular function measurement control unit 4248 determines the displacement of the blood vessel diameter based on the positions of the front wall and the rear wall of the blood vessel 4004 which are continuously measured by the tracking unit 4210, and performs the control relevant to predetermined vascular function measurement. For example, the operation control of executing processing of estimating a blood pressure from the blood vessel diameter using a stiffness parameter and measuring the blood pressure is performed, for example.

The measurement result record and display control unit 4250 performs control for storing the measurement result of the vascular function in the storage unit 4500 and displaying the measurement result on the display unit 4300.

The image generation unit 4260 generates an image for displaying a measurement result or various operation screens required for ultrasonic measurement or biological information measurement, and outputs the image to the display unit 4300.

The display unit 4300 displays image data input from the image generation unit 4260. The touch panel 4012 shown in Fig. 26 corresponds to the display unit.

The storage unit 4500 is realized by a storage medium, such as an IC memory, a hard disk, or an optical disc, and stores various programs or various kinds of data, such as data in the operation process of the processing unit 4200. In Fig. 26, the storage medium 4033 mounted in the control board 4031 of the processor 4030 corresponds to the storage unit. In addition, the connection between the processing unit 4200 and the storage unit 4500 is not limited to a connection using an internal bus circuit in the apparatus, and may be realized by using a communication line, such as a local area network (LAN) or the Internet. In this case, the storage unit 4500 may be realized by using a separate external storage device from the ultrasonic measurement apparatus 4010.

In addition, as shown in Fig. 31, the storage unit 4500 stores a measurement program 4501, reflected wave data 4510, setting data 4520, conformance condition data 4530, correlation operation result data 4550, and vascular function measurement data 4570. Needless to say, frame identification information, various flags, counter values for time checking, and the like other than those described above can also be appropriately stored.

The processing unit 4200 realizes the functions of the ultrasonic wave control unit 4202, the tracking initial control unit 4230, the blood vessel walls detection unit 4244, the vascular function measurement control unit 4248, the measurement result record and display control unit 4250, and the image generation unit 4260, by reading out and executing the measurement program 4501. The tracking initial control program 4502 for realizing the function of the tracking initial control unit 4230 is included in the measurement program 4501 as a subroutine program.

In addition, when realizing these functional units with hardware, such as an electronic circuit, a part of the program for realizing the function can be omitted.

The reflected wave data 4510 is reflected wave data obtained by ultrasonic measurement, and is generated for each frame by the ultrasonic wave control unit 4202. A piece of reflected wave data 4510, for example, includes a scanning line ID 4512, a measurement frame 4514, and received signal data (depth-signal strength data) 4516. Needless to say, data other than those described above can also be appropriately stored.

The setting data 4520 stores the signal waveform template 4523 set by the template setting unit 4236, and the data of the searching range 4525 for searching the signal parts set as the tracking point by the searching unit 4238 from the received signals at the second timing when the measurement is started, based on the received signals at the first timing. The searching range 4525 is, for example, set as the range relative to the depth position as a reference, by the tracking starting control unit 4237. The depth position as a reference can be set as a position of the signal waveform template 4523 among the received signals at the first timing.

The conformance condition data 4530 is data for determining the conditions for the correlation values for determining the signal part set as the tracking point by the searching unit 4238.

The correlation operation result data 4550 is data in which the correlation values acquired by the correlation operation of the correlation operation unit 4239 is stored by correlating to the position selected as the operation target signal part.

The vascular function measurement data 4570 stores the data in which the positions of the front wall and the rear wall of the blood vessel 4004 tracked by the tracking unit 4210 correspond to the time information at which the corresponding received signal is obtained, the data of the blood vessel diameter calculated from the positions of the front wall and the rear wall, or the data of the blood pressure calculated based on the blood vessel diameter.

### Description of Flow of Process of Fourth Embodiment

Next, the operation of the measurement process of the ultrasonic measurement apparatus 4010 will be described.

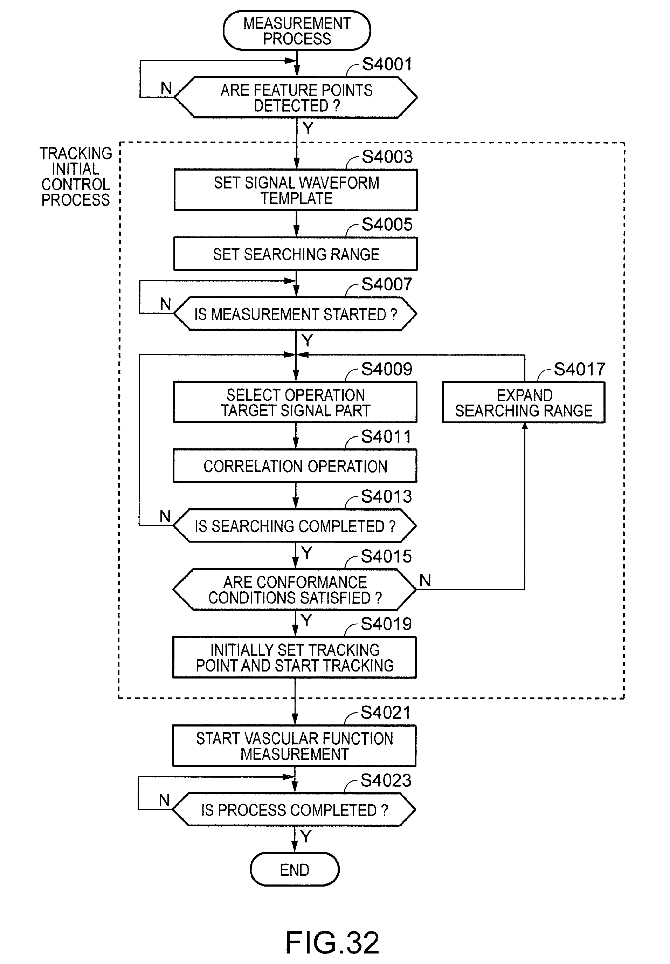

Fig. 32 is a flowchart for illustrating a flow of measurement process of the ultrasonic measurement apparatus 4010 in the fourth embodiment.

In this process, the processing unit 4200 starts a process of transmitting an ultrasonic beam for each ultrasonic transducer (scanning line) of the ultrasonic wave transmission and reception unit 4102, receiving the reflected wave, and storing the reflected wave data 4510 in the storage unit 4500 on each occasion.

First, the blood vessel walls detection unit 4244 determines whether or not the feature points are included in the received signal, based on the received signal data 4516 obtained by the transmission and reception of the ultrasonic waves on each occasion, and therefore stands by until the feature points are detected (Step S4001: No). In the fourth embodiment, the feature points are peak indicating the front wall and the rear wall of the blood vessel 4004. When the feature points are detected (Step S4001: YES), the tracking initial control process is performed by the tracking initial control unit 4230 (Steps S4003 to S4019).

That is, the template setting unit 4236 sets the signal waveform template using the received signal obtained at the first timing (Step S4003). Then, the tracking starting control unit 4237 sets the searching range 4525 (Step S4005). The searching unit stands by until the instruction to start the measurement is operated (Step S4007: NO), and when the instruction is operated (Step S4007: YES), the searching unit 4238 selects the operation target signal part from the searching range 4525 among the received signals obtained at the second timing which is the timing when the instruction is operated (Step S4009). The correlation operation unit 4239 performs the correlation operation of the operation target signal part and the signal waveform template 4523 (Step S4011), and stores the acquired correlation value in the correlation operation result data 4550 by correlating with the position selected as the operation target signal part.

When the execution of the processes in Steps S4009 to S4011 by deviating the selected position as the operation target signal parts in the searching range 4525 is completed, the searching ends (Step S4013: YES), and it is determined whether or not the correlation value is a correlation value satisfying the conformance conditions from the correlation operation result data 4550 (Step S4015). When there is no satisfied correlation value, the processes in Step S4009 to s4013 are repeated again by expanding the searching range 4525 (Step S4017).

When there is the correlation value satisfying the conformance conditions, the tracking starting control unit 4237 initially sets the tracking point in the position of the signal part corresponding to the correlation value among the signal parts obtained at the second timing and controls the tracking unit 4210 to start the tracking (Step S4019).

By starting the tracking after the second timing by the tracking unit 4210, the measurement of the vascular function is started by the vascular function measurement control unit 4248 (Step S4021).

After that, the measurement is continued until the operation of completion of the measurement process is performed, and when the completion operation is performed (Step S4023: YES), the measurement process ends.

Hereinabove, according to the fourth embodiment, the signal waveform template showing the part of the blood vessel wall of the blood vessel is set based on the received signal obtained at the first timing, among the received signals obtained by receiving the reflected wave of the ultrasonic wave transmitted to the blood vessel 4004. Thus, the setting of the signal waveform template can be performed based on the received signal at a certain time, as a still image, and therefore it is possible to accurately set the desired tracking target.

After the setting of the signal waveform template, the signal part conformable to the signal waveform template is searched, the tracking point is set in the part, and the tracking is started. Accordingly, although there is a difference in time between the timing when the signal waveform template is set and the starting timing of the tracking, the part desired to be tracked and the part to actually be tracked are not different from each other, regardless of the problems regarding the difference in time.

In the fourth embodiment, it is possible to appropriately add, omit, and change the constituent elements. For example, in the fourth embodiment, the both of the front wall and the rear wall of the blood vessel are the tracking targets in the above description, but only one thereof may be the tracking target.

In addition, the correlation operation is performed in the above description, but instead of the similarity such as the correlation value, dissimilarity such as sum of squared difference (SSD) or sum of absolute difference (SAD) may be used. However, when using SSD or SAD, as the value decreases the similarity increases, and therefore the equivalent conditions (threshold value condition) are set to have the value equal to or smaller than the threshold value.

As described above, the embodiments of the invention have been described in detail, but it is apparent to a person skilled in the art that various modifications substantially not departing from novel matters and effects of the invention can be performed. Therefore, all of modification examples are included in the scope of the invention.

## Claims

1. An ultrasonic measurement apparatus comprising:
a transmission and reception control unit which controls transmission of an ultrasonic wave toward a blood vessel and reception of a reflected wave;
a setting unit which sets a signal range including a signal part for tracking movement of blood vessel walls of the blood vessel due to pulsation, and a signal waveform template in the signal range, among received signals of the reflected waves obtained at a first timing;
a detection unit which detects a second timing when a signal waveform of the signal part corresponding to the signal range among the received signals and the signal waveform template satisfy predetermined equivalent conditions; and
a tracking unit which starts the tracking at the second timing.

2. The ultrasonic measurement apparatus according to claim 1,
wherein the setting unit sets a front wall signal range and a front wall signal waveform template relating to a signal part of a front wall of the blood vessel, and a rear wall signal range and a rear wall signal waveform template relating to a signal part of a rear wall, and
the detection unit detects a timing when a signal waveform of the signal part corresponding to the front wall signal range and the front wall signal waveform template satisfy the equivalent conditions and a signal waveform of the signal part corresponding to the rear wall signal range and the rear wall signal waveform template satisfy the equivalent conditions, as the second timing.

3. An ultrasonic measurement method comprising:
controlling transmission of an ultrasonic wave toward a blood vessel and reception of a reflected wave;
setting a signal range including a signal part for tracking movement of blood vessel walls of the blood vessel due to pulsation, and a signal waveform template in the signal range, among received signals of the reflected waves obtained at a first timing;
detecting a second timing when a signal waveform of the signal part corresponding to the signal range among the received signals and the signal waveform template satisfy predetermined equivalent conditions; and
starting the tracking at the second timing.

4. The ultrasonic measurement apparatus according to claim 1,
wherein the setting unit sets a front wall signal range relating to a signal of a front wall of the blood vessel and a rear wall signal range relating to a signal of a rear wall, and
the detection unit detects a timing when the signal corresponding to the front wall signal range satisfies the conditions and the signal corresponding to the rear wall signal range satisfies the conditions, as a second timing.

5. The ultrasonic measurement apparatus according to claim 4,
wherein the predetermined conditions are conditions based on any one of a total amplitude value, an average amplitude value, and a mean amplitude value of the signals corresponding to the signal ranges.

6. The ultrasonic measurement apparatus according to any of claims 1, 2, 4 or 5,
wherein the timing detection unit detects the first timing by tracking the received signals of the reflected waves in advance.

7. The ultrasonic measurement apparatus according to any of claims 1, 2, 4, 5 or 6,
wherein the timing detection unit detects a timing when displacement of peak waveform parts included in the received signals satisfies predetermined conditions, as the first timing.

8. The ultrasonic measurement apparatus according to any of claims 1, 2 or 4 to 7,
wherein the timing detection unit detects the first timing based on the displacement of the peak waveform parts shown in a position of an external film part of the blood vessel.

9. The ultrasonic measurement apparatus according to any of claims 1, 2 or 4 to 8,
wherein the timing detection unit detects the first timing by measuring a change in a blood vessel diameter of the blood vessel by the tracking in advance.

10. The ultrasonic measurement apparatus according to any of claims 1, 2 or 4 to 9,
wherein the transmission and reception unit performs the reception at a predetermined frame rate, and
the timing detection unit detects a timing when correlation values obtained by a correlation operation of the received signals in preceding and succeeding frames satisfy predetermined conditions, as the first timing.

11. The ultrasonic measurement apparatus according to any of claims 1, 2 or 4 to 10,
wherein the timing detection unit detects a timing of a telediastolic period from the diastole, as the first timing.

12. The ultrasonic measurement apparatus according to any of claims 1, 2 or 4 to 11,
wherein the setting unit sets a front wall signal range and a front wall signal waveform template relating to a signal part of a front wall of the blood vessel, and a rear wall signal range and a rear wall signal waveform template relating to a signal part of a rear wall, and
the detection unit detects a timing when a signal waveform of the signal part corresponding to the front wall signal range and the front wall signal waveform template satisfy the conditions and a signal waveform of the signal part corresponding to the rear wall signal range and the rear wall signal waveform template satisfy the conditions, as the second timing.

13. The ultrasonic measurement apparatus according to any of claims 1, 2 or 4 to 12,
wherein the blood vessel is an artery.
